(19) Europäisches Patentamt
European Patent Office
Office européen des brevets

(11) EP 3 207 864 A1

(12) **EUROPEAN PATENT APPLICATION**

(43) Date of publication:
**23.08.2017 Bulletin 2017/34**

(21) Application number: **17153710.3**

(22) Date of filing: **30.01.2017**

(51) Int Cl.:
***A61B 5/024*** *(2006.01)* ***A61B 5/08*** *(2006.01)*
***A61B 5/113*** *(2006.01)* ***A61B 5/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.02.2016 JP 2016027971**

(71) Applicant: **FUJITSU LIMITED**
**Kawasaki-shi Kanagawa 211-8588 (JP)**

(72) Inventor: **Yamaji, Takayuki**
**Kawasaki-shi Kanagawa 211-8588 (JP)**

(74) Representative: **Hoffmann Eitle**
**Patent- und Rechtsanwälte PartmbB**
**Arabellastraße 30**
**81925 München (DE)**

(54) **SENSOR INFORMATION PROCESSING APPARATUS**

(57) A filter to cut at least a harmonic component of a frequency component derived from breathing of a person to be observed is applied to a detected signal of a wireless sensor (21), and a heartbeat component of the person is detected in a signal having passed through the filter.

FIG. 1

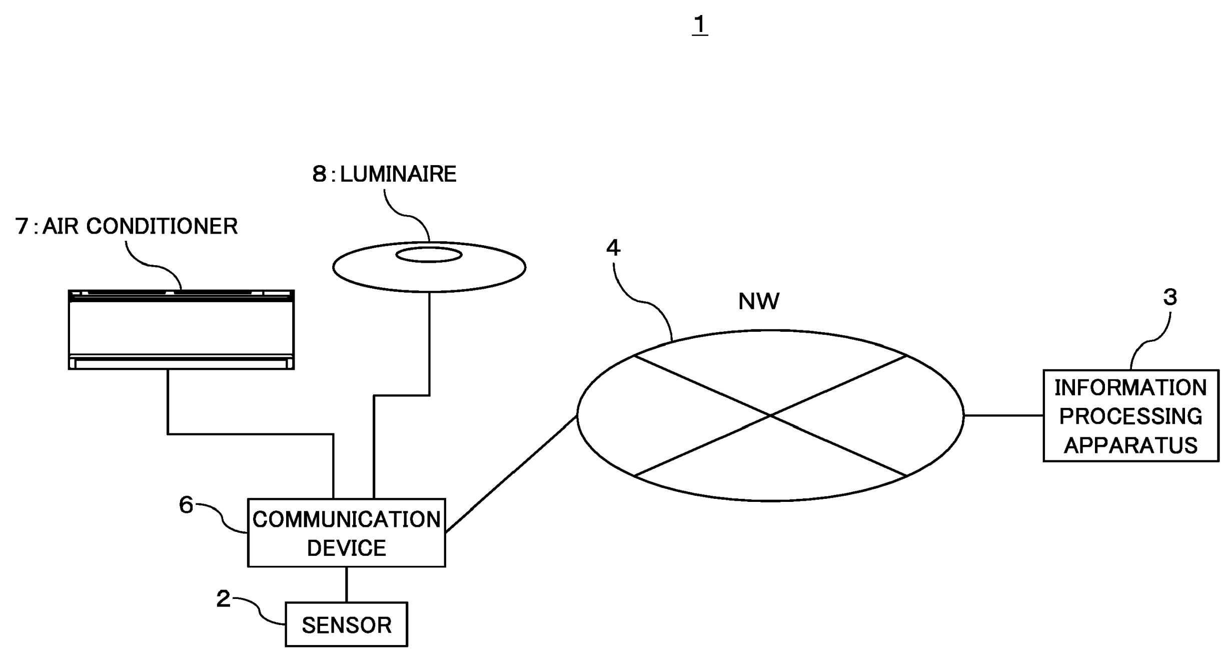


EP 3 207 864 A1

## Description

FIELD

The embodiment(s) discussed herein is related to a sensor information processing apparatus.

BACKGROUND

There is a technique of detecting a heartbeat of a biological object by using a wireless sensor. A bandpass filter (BPF) is applied to a detected signal of the wireless sensor to detect a signal component corresponding to the heartbeat from the detected signal of the wireless sensor.

(RELATED ART DOCUMENTS LIST)

Patent Document 1 JP2014-39666 A
Patent Document 2 JP1-115344 A
Patent Document 3 JP2011-115459 A
Patent Document 4 JP2008-125595 A

When a heart rate is higher, the breathing rate of a human body which is an example of a biological object also tends to be higher. When the breathing rate is higher, for example, a motion of a breast of the human body tends to be greater accompanying breathing.

Hence, in addition to a signal component corresponding to a heartbeat of the human body, a signal component corresponding to a motion of the breast is likely to be mixed in a detected signal of the wireless sensor.

The signal component corresponding to the motion of the breast becomes a noise component with respect to a target heartbeat derived signal component (which may be referred to as a "heartbeat component" or a "heartbeat signal") to be detected in a detected signal of the wireless sensor.

While the noise component can be suppressed by applying a BPF, when the breathing rate becomes higher, a harmonic component of a frequency component derived from breathing is mixed in a passband of the BPF in the detected signal of the wireless sensor in some cases.

The harmonic component also tends to become a noise component with respect to a heartbeat component, and, when the breathing rate becomes higher, heartbeat component detection precision lowers.

SUMMARY

According to one aspect, one of objects of techniques discussed herein is to suppress at least a harmonic component of a frequency component derived from breathing in a detected signal of a wireless sensor, and improve a detection accuracy of a heartbeat component.

In one aspect, a sensor information processing apparatus may include a receiver and a processor. The receiver may receive a detected signal of a wireless sensor. The processor may apply to the detected signal a filter configured to cut at least a harmonic component of a frequency component derived from breathing of a person to be observed, and detect a heartbeat component of the person in a signal having passed through the filter.

Further, in another aspect, a sensor information processing apparatus may include a receiver and a processor. The receiver may receive a detected signal of a wireless sensor and a detected signal of an inertial sensor. The processor may control a cutoff frequency of a high-pass filter applied to the detected signal of the wireless sensor, according to the detected signal of the inertial sensor, and detect a heartbeat component of a person to be observed in a signal having passed through the high-pass filter.

According to one aspect, it is possible to suppress at least a harmonic component of a frequency component derived from breathing in a detected signal of a wireless sensor, and improve a detection accuracy of a heartbeat component.

BRIEF DESCRIPTION OF DRAWINGS

FIG. 1 is a block diagram illustrating an example of a sensor system according to an embodiment;

FIGS. 2 and 3 are block diagrams illustrating configuration examples of a vital sensor according to an embodiment;
FIG. 4 is a block diagram illustrating a configuration example of an information processing apparatus according to an embodiment;
FIG. 5 is a diagram illustrating an example of a result obtained by analyzing a frequency of a wireless sensor value according to one embodiment;
FIG. 6 is a diagram illustrating a characteristics example of a high-pass filter (HPF) according to one embodiment;
FIG. 7 is a flowchart illustrating an operation example of a sensor system according to a first embodiment;
FIG. 8 is a diagram illustrating an example of a heart rate measurement result according to one embodiment;
FIG. 9 is a diagram illustrating an example of signal waveforms according to one embodiment in a frequency domain of a wireless sensor value in case where the HPF is applied to a wireless senor value and in case where the HPF is not applied to wireless sensor value;
FIG. 10 is a flowchart illustrating an operation example of a sensor system according to a second embodiment;
FIG. 11 is a flowchart illustrating an operation example of a sensor system according to a third embodiment;
FIG. 12 is a flowchart illustrating a first example of a heart rate estimation process illustrated in FIG. 11;
FIG. 13 is a diagram illustrating an example of a relation between a walking cadence and a heart rate according to one embodiment;
FIG. 14 is a flowchart illustrating a second example of the heart rate estimation process illustrated in FIG. 11;
FIG. 15 is a diagram illustrating an example of a relation between a walking cadence and a walking speed according to one embodiment;
FIG. 16 is a diagram illustrating an example of a relation between a walking speed and an exercise intensity (METs value) according to one embodiment;
FIG. 17 is a diagram illustrating an example of a heart rate and the breathing rate associated with a walking speed according to one embodiment;
FIG. 18 is a diagram illustrating an example of a relation between a heart rate and a breathing rate according to one embodiment;
FIG. 19 is a diagram illustrating a setting example of a BPF according to one embodiment;
FIG. 20 is a diagram illustrating an example of a passband of the BPF according to one embodiment;
FIG. 21 is a diagram illustrating a setting example of a bandwidth of the BPF according to one embodiment;
FIGS. 22 and 23 are diagrams illustrating examples of distributions of the heart rate according to an embodiment;
FIGS. 24 and 25 are diagrams illustrating examples of a statistical process of the heart rate according to an embodiment;
FIGS. 26 and 27 are diagrams illustrating setting examples of a bandwidth of the BPF according to one embodiment; and
FIG. 28 is a diagram illustrating an example of bandwidth information of the BPF according to an embodiment.

## DESCRIPTION OF EMBODIMENTS

Hereinafter, an exemplary embodiment (s) will be described with reference to the drawings. However, the embodiment(s) described below is merely an example and not intended to exclude an application of various modifications or techniques which are not explicitly described below. Further, various exemplary aspects described below may be appropriately combined and carried out. Elements or components assigned the same reference numeral in the drawings used for the following embodiment(s) will represent identical or similar elements or components unless otherwise specified.

FIG. 1 is a block diagram illustrating an example of a sensor system according to an embodiment. A sensor system 1 illustrated in FIG. 1 may illustratively include a vital sensor 2, an information processing apparatus 3 and a network (NW) 4.

The vital sensor 2 may illustratively be connected to the network 4 through a communication device 6, and may be available to communicate with the information processing apparatus 3 through the network 4.

The vital sensor 2 is available to sense information of a biological object (hereinafter, may be referred to as "vital information"). The biological object is an example of a sensing target. The term of "sensing" may be referred to as "detection" or "measurement".

A non-restrictive example of the "vital information" is information indicating a heartbeat, breathing or a motion of a biological object. The "biological object" may include an "organ" of the biological object. A "heartbeat" may be regarded as information indicating a motion of a "heart" which is an example of the "organ".

The motion of the biological object (which may be paraphrased as a "position change") may be abbreviated as a "body motion" for descriptive purposes. The "body motion" may illustratively include not only a motion during an activity of the biological object but also a motion of a biological object surface (e.g. skin) associated with a heartbeat or breathing during a rest time such as a sleeping time of the biological object.

The motion of the biological object surface may be regarded to occur in response to a motion of the organ of the biological object. For example, the skin makes a motion in response to a heartbeat. Further, the skin makes a motion in response to stretching and contraction of lungs accompanying breathing. In addition, a vein of the biological object pulsates according to a heartbeat, and therefore the information indicating the heartbeat may be regarded as equivalently as information indicating a pulse.

The vital sensor 2 can illustratively irradiate a sensing target with a radio wave such as a microwave, and detect the "motion" of the biological object based on a change in a reflected wave reflected by the sensing target and received without contacting the biological object. Hence, the vital sensor 2 may be referred to as the "non-contact vital sensor 2" for descriptive purposes.

When, for example, a distance between the vital sensor 2 (which is also abbreviated simply as the "sensor 2" below) and the sensing target changes, the reflected wave changes due to a Doppler effect. The change in the reflected wave can be illustratively regarded as changes in one or both of an amplitude and a frequency of the reflected wave.

The sensor 2 may be attached to a human body which is an example of the biological object. An attachment position of the sensor 2 with respect to the human body may be illustratively a breast of the human body. For example, a distance between the sensor 2 attached to the human body, and the skin and the heart of the human body changes accompanying a beat of the human body.

Hence, the change corresponding to the change in the distance appears in the reflected wave of a radio wave irradiated by the sensor 2. For example, it is possible to measure, for example, a heartbeat or a pulse of the human body based on the change in the reflected wave.

The sensor 2 which can measure a heartbeat may be referred to as a "heartbeat sensor" or a "heartbeat monitor". As described above, since the information indicative of a "heartbeat" may be considered as being equivalent to the information indicative of a "pulsebeat" in some cases, the "heartbeat sensor" or the "heartbeat meter" may be referred to as a "pulsebeat sensor" or a "pulsebeat meter".

The sensor 2 may be illustratively attached in contact with a skin of a human body or may be attached to clothes of the human body. The sensor 2 does not need to be attached to a human body by way of fixing (which may be referred to as "restrain"), strictly saying. It may be allowed to occur a relative motion between the vital sensor 2 and a human body according to a mismatch between motions of the clothes and a human body surface.

For example, the sensor 2 may be attached to a human body such that the vital sensor 2 is allowed to be moved in one of three-dimensional directions relative to a human body. Illustratively, the sensor 2 may be put in a pocket of clothes such as a breast pocket of a jacket or may be attached on the clothes by using an attachment tool such as a harness.

For example, the vital sensor 2 may be attached to a human body such that the vital sensor 2 is allowed to be moved in one of three-dimensional directions relative to a human body. Illustratively, the vital sensor 2 may be put in a pocket of clothes such as a breast pocket of a jacket or may be attached on the clothes by using an attachment tool such as a harness.

Next, the communication device 6 illustrated in FIG. 1 is available to transmit a sensing result (e.g. the information indicative of the heartbeat) of the vital sensor 2 to the information processing apparatus 3 through the network 4, for example. Hence, the communication device 6 may be connected with the network 4 using a wired cable or radio.

In other words, the communication device 6 may be provided with a communication interface (IF) which supports one or both of wireless and wired communications. Illustratively, a communication scheme based on the LTE (Long Term Evolution) or the LTE-Advanced of 3GPP (3rd Generation Partnership Project) is applicable to the wireless communication of the communication device 6.

Further, satellite communications may be applied to the wireless communication of the communication device 6. When the satellite communication is applied, the communication device 6 may be able to communicate with the information processing apparatus 3 through a communication satellite without being routed through the network 4.

The sensing result of the vital sensor 2 may include not only vital information but also information indicative of a result of an arithmetic operation or determination, which is obtained based on the vital information. The sensing result may be referred to as "sensor information" or "sensor data" for descriptive purposes.

The communication device 6 may be externally attached to the vital sensor 2 as illustrated in FIG. 1, or may be built in the vital sensor 2. The communication device 6 externally attached to the vital sensor 2 may be, for example, a device carried by a person attached with the vital sensor 2. The person attached with the vital sensor 2 may be referred to as a "user", a "subject" or an "observed person" for descriptive purposes.

The communication device 6 carried by the user may be illustratively a mobile telephone (which may include a smartphone), a notebook PC or a tablet PC. The "PC" is an abbreviation of a "personal computer".

A wired connection or a wireless connection may be applied to a connection between the vital sensor 2 and the communication device 6. In other words, the vital sensor 2 may be provided with a communication IF which supports one or both of wireless and wired communications. The "WiFi (Wireless Fidelity)" (registered trademark) or "Bluetooth" (registered trademark) may also be applied to the wireless connection.

The communication device 6 externally attached to the vital sensor 2 may be a router or a network switch. As illustrated in FIG. 1, the communication device 6 may be communicably connected with an air conditioner 7 and an luminaire 8 so that the air conditioner 7 and the luminaire 8 are able to communicate with the information processing apparatus 3 through the network 4.

The network 4 may be illustratively a WAN (Wide Area Network), a LAN (Local Area Network) or the Internet. Further, the network 4 may include a wireless access network. The wireless access network may be compliant with the above-described LTE or LTE-Advanced.

The information processing apparatus 3 receives the sensor information of the vital sensor 2 through the network 4 (or may be through the communication satellite), and processes the received sensor information. Hence, the information processing apparatus 3 may be referred to as the sensor information processing apparatus 3.

Processing the sensor information may include storing and managing the sensor information, and estimating a heart rate of the user based on the sensor information. Hence, the information processing apparatus 3 is available to monitor an activity status of the user, for example. In other words, the sensor system 1 can provide a user "monitoring (or watching) function".

Managing the sensor information may include compiling the sensor information in a database (DB). The data compiled in the DB may be referred to as "cloud data" or "big data".

The information processing apparatus 3 may be illustratively realized by one or a plurality of servers. In other words, the sensor information obtained by the vital sensor 2 may be processed or managed by a single server or may be distributedly processed or managed by a plurality of servers in the information processing apparatus 3. The server may correspond to a cloud server provided in a cloud data sensor, for example.

The information processing apparatus 3 may be communicably connected with the vital sensor 2 without being routed through the network 4. For example, the information processing apparatus 3 may be available to directly receive the sensor information from the vital sensor 2 through a wired cable or by radio.

(Configuration example of vital sensor 2)

Next, the configuration example of the vital sensor 2 will be described with reference to FIGS. 2 and 3. As illustrated in FIGS. 2 and 3, the vital sensor 2 may illustratively include a wireless sensor 21, a processor 23, a memory 24 and a communication IF 25.

In addition, the vital sensor 2 may additionally include an optional inertial sensor 22 as indicated by dotted lines in FIGS. 2 and 3. The vital sensor 2 may be referred to as the sensor unit 2. Hereinafter, the vital sensor 2 or the sensor unit 2 will be simply referred to as the "sensor 2" for descriptive purposes.

As illustrated in FIG. 3, the wireless sensor 21, the processor 23, the memory 24 and the communication IF 25 may be illustratively connected to a bus 26 to communicate with each other through the processor 23. The optional inertial sensor 22 may be also connected to the bus 26.

The wireless sensor 21 is an example of a heartbeat sensor which can measure a heartbeat by using the Doppler effect. The "wireless sensor" may be referred to as a "microwave sensor", a "RF (Radio Frequency) sensor" or a "Doppler sensor". The wireless sensor 21 may perform phase detection on a radio wave transmitted to a space and a reflected wave of the transmitted radio wave, and generate a beat signal. The beat signal may be supplied as an output signal of the wireless sensor 21 to the processor 23.

As illustrated in FIG. 2, the wireless sensor 21 may include, for example, an antenna 211, a local oscillator (OSC) 212, a MCU (Micro Control Unit) 213, a detection circuit 214, an operational amplifier (OP) 215 and a power supply unit (or a power supply circuit) 216.

The antenna 211 transmits to the space a radio wave having an oscillation frequency generated by the OSC 212, and receives a reflected wave of the transmitted radio wave reflected by the user positioned in the space. In an example of FIG. 2, the antenna 211 is shared by a transmission and a reception but a transmission antenna and a reception antenna may be provided individually.

The OSC 212 illustratively oscillates in response to a control of the MCU 213 to output a signal with a predetermined frequency (which may be referred to as a "local signal" for descriptive purposes). The local signal is transmitted as a transmission radio wave from the antenna 211, and is inputted to the detection circuit 214.

The oscillation frequency of the OSC 212 (in other words, a frequency of a radio wave transmitted by the wireless sensor 21) may be illustratively a frequency in a microwave band. The microwave band may be illustratively a 2.4 GHz band or a 24 GHz band.

These frequency bands are examples whose indoor use is authorized by the Radio Act in Japan. Frequency bands which are not regulated by the Radio Act may be used for a transmission radio wave of the wireless sensor 21.

The MCU 213 illustratively controls an oscillating operation of the OSC 212 in response to a control of the processor 23.

The detection circuit 214 illustratively performs the phase detection on the reflected wave received by the antenna 211 and the local signal (in other words, the transmission radio wave) from the OSC 212 to output the beat signal. The detection circuit 214 may be replaced with a mixer which mixes a transmission radio wave and a reflected wave. The mixing performed by the mixer may be considered as being equivalent to the phase detection.

In this regard, a change in an amplitude and a change in a frequency occur in the beat signal obtained by the detection circuit 214 due to the Doppler effect according to a heartbeat of the user. In other words, the beat signal includes information indicative of the heartbeat of the user.

The operational amplifier 215 illustratively amplifies the beat signal outputted from the detection circuit 214. The amplified beat signal is inputted to the processor 23.

The power supply unit 216 illustratively supplies drive power to the MCU 213, the detection circuit 214 and the operational amplifier 215.

The optional inertial sensor 22 may illustratively detect a motion of the sensor unit 2. The inertial sensor 22 may be an acceleration sensor or a gyroscope. Any one of piezoelectric type and capacitive type sensors may be illustratively applied to the acceleration sensor. Any one of a spin rotor (flywheel) type, an optical type and a vibrating structure type sensors may be applied to the gyroscope.

The inertial sensor 22 may include one or more of detection axes. A gravity component in a direction along one of the detection axes may be detected as an "acceleration" component, for example. A detected signal of the inertial sensor 22 may be inputted to the processor 23.

The processor 23 is an example of an arithmetic processing apparatus with a capability of arithmetic processing. The arithmetic processing apparatus may be referred to as an arithmetic processing device or an arithmetic processing circuit. An integrated circuit (IC) such as an MPU (Micro Processing Unit) or a DSP (Digital Signal Processor) may be illustratively applied to the processor 23 which is an example of the arithmetic processing apparatus. The "processor" may be referred to as a "processing unit", a "controller" or a "computer".

The processor 23 is available to detect the heartbeat of the user based on the detected signal of the wireless sensor 21. A filter may be illustratively applied to the detected signal of the wireless sensor 21 to detect a heartbeat derived signal component (which may be referred to as a "heartbeat component" or a "heartbeat signal") from the detected signal of the wireless sensor 21.

A high-pass filter (HPF) and a bandpass filter (BPF) may be applied to the filter of a non-restrictive example. The HPF may be referred to as a "low-cut filter (LCF)". In this regard, the BPF may be optional.

The processor 23 may determine a state related to a sleep of the user based on the detected heartbeat.

When the sensor 2 includes the inertial sensor 22, a detected signal of the inertial sensor 22 may be used to control filter characteristics of the HPF and the BPF. The filter characteristics of the HPF may be referred to as "HPF characteristics" for descriptive purposes, and the filter characteristics of the BPF may be referred to as "BPF characteristics" for descriptive purposes.

Controlling the HPF characteristics may illustratively mean controlling a cutoff frequency (which may be referred to as a "cut-off frequency"). A signal having a lower frequency than the cut-off frequency is reduced or suppressed, and a signal having a higher frequency than the cut-off frequency is hardly attenuated. A frequency band lower than the cut-off frequency will be referred to as a "cutoff band" of the HPF for descriptive purposes.

Controlling the BPF characteristics may illustratively mean controlling at least one of a pass center frequency and a passband width of the BPF. The "pass center frequency" and the "passband width" of the BPF may be referred to simply as a "center frequency" and a "bandwidth", respectively.

Controlling the HPF characteristics and the BPF characteristics may be regarded to mean controlling a target frequency band (or a frequency band excluded from a target to be processed) to be processed in the detected signal of the wireless sensor 21. An example of filter characteristics control will be described below.

The detected signal of the wireless sensor 21 and the detected signal of the inertial sensor 22 may be both referred to as a "detected value" or an "output value". For descriptive purposes, the detected value of the wireless sensor 21 may be referred to as a "wireless sensor value" and the detected value of the inertial sensor 22 may be referred to as an "inertial sensor value".

Further, the above-described detection of the heartbeat and control of the filter characteristics may be performed by a processor 31 of the information processing apparatus 3 (described below with reference to FIG. 4) instead of the processor 23 of the sensor unit 2.

Next, in FIG. 3, the memory 24 is an example of a storage unit or a storage medium provided in the sensor unit 2, and may be a RAM (Random Access Memory) or a flash memory.

A program and data read and used by the processor 23 to operate may be stored in the memory 24. The "program" may be referred to as a "software" or an "application". The "data" may include data generated according to operations of the processor 23.

The communication IF 25 is an example of a communication unit of the sensor unit 2, and is illustratively connected with the communication device 6 (see FIG. 1) and enables communication with the information processing apparatus 3 through the network 4.

For example, the communication IF 25 may transmit the detected signal of the wireless sensor 21 or each detected signal of the wireless sensor 21 and the inertial sensor 22 to the information processing apparatus 3. Signals and information transmitted to the information processing apparatus 3 are not limited to detected signals and may be information obtained based on the detected signals.

In other words, the sensor information transmitted from the vital sensor 2 to the information processing apparatus 3 may include each measured value of the wireless sensor 21 (or each measured value of the wireless sensor 21 and the inertial sensor 22), or may include information obtained based on the measured value.

The communication IF 25 may be connected with the information processing apparatus 3 to directly communicate with the information processing apparatus 3 without being routed through the communication device 6 and/or the network 4.

(Configuration example of information processing apparatus 3)

Next, the configuration example of the information processing apparatus 3 illustrated in FIG. 1 will be described with reference to FIG. 4. As illustrated in FIG. 4, the information processing apparatus 3 may illustratively include the processor 31, a memory 32, a storage device 33, a communication interface (IF) 34 and a peripheral IF 35.

The processor 31, the memory 32, the storage device 33, the communication IF 34 and the peripheral IF 35 may be illustratively connected via a bus 36 to communicate with each other via the processor 31.

The processor 31 is an example of an arithmetic processing apparatus which has a capability of arithmetic processing. The arithmetic processing apparatus may be referred to as an arithmetic processing device or an arithmetic processing circuit. An IC such as a CPU or a MPU or a DSP may be illustratively applied to the processor 31 which is an example of the arithmetic processing apparatus. The "processor" may be referred to as a "processing unit", a "controller" or a "computer".

The processor 31 illustratively controls an entire operation of the information processing apparatus 3. The control performed by the processor 31 may include a control for communication performed through the network 4. By controlling the communication, the air conditioner 7 and the luminaire 8 may be remotely controlled through the network 4, for example.

Illustratively, the processor 31 may detect the heartbeat or control the filter characteristics as described above based on the sensor information of the vital sensor 2, which is received by the communication IF 34.

Further, the processor 31 may illustratively generate a control signal to control a spatial environment in which the user of the vital sensor 2 is positioned, such as a control signal to control operations of the air conditioner 7 and the luminaire 8.

The control signal may be illustratively generated based on a heart rate of the user detected based on the sensor information obtained from the vital sensor 2 and a state related to a sleep of the user estimated or determined based on the heart rate.

The control signal generated by the processor 31 may be illustratively transmitted to the air conditioner 7 and the luminaire 8 through the communication IF 34.

The memory 32 is an example of a storage medium, and may be a RAM or a flash memory. A program and data read and used by the processor 31 to operate may be stored in the memory 32.

The storage device 33 may store various pieces of data and programs. A hard disk drive (HDD), a solid state drive (SSD) or a flash memory may be applied to the storage device 33.

The data stored in the storage device 33 may illustratively include the sensor information of the sensor 2 received by the communication IF 34, the heart rate detected based on the sensor information, and the state related to the sleep of the user estimated or determined based on the heart rate.

The data stored in the storage device 33 may be optionally compiled in a database (DB). The data compiled in the DB may be referred to as "cloud data" or "big data". The storage device 33 and the memory 32 may be collectively referred to as a "storage unit 30" of the information processing apparatus 3.

The programs stored in the storage device 33 may include a program to execute processes described with reference to FIGS. 7, 10 and 11.

The program to execute the processes described below with reference to FIGS. 7, 10 and 11 may be referred to as a "sensor information processing program" for descriptive purposes.

All or part of program codes configuring a program may be stored in the storage unit or may be described as a part of an operating system (OS).

The program and the data may be recorded in a computer-readable non-transitory recording medium to be provided. Examples of the recording medium include flexible disks, CD-ROMs, CD-Rs, CD-RWs, MOs, DVDs, Blu-ray disks and portable hard disks. Further, a semiconductor memory such as a USB (Universal Serial Bus) memory is an example of a recording medium.

Alternatively, the program and the data may be provided (or downloaded) from the server to the information processing apparatus 3 through the network 4. For example, the program and the data may be provided to the information processing apparatus 3 through the communication IF 34. Further, the program and the data may be inputted to the information processing apparatus 3 from an input device described below which is connected to the peripheral IF 35.

The communication IF 34 is an example of a communication unit provided in the information processing apparatus 3, and is illustratively connected to the network 4 to enable communication through the network 4.

Upon focusing on a reception process, the communication IF 34 is an example of a receiver (which may be referred to as an "acquiring unit") which receives information transmitted from the vital sensor 2 to the information processing apparatus 3.

Meanwhile, upon focusing on a transmission process, the communication IF 34 is an example of a transmitter which transmits the control signal generated by the processor 31 to the vital sensor 2, the air conditioner 7 and the luminaire 8, for example. An Ethernet (registered trademark) card may be illustratively applied to the communication IF 34.

The communication IF 34 may be connected with the communication IF 25 of the vital sensor 2 without being routed through the network 4 to enable direct communication with the vital sensor 2.

The peripheral IF 35 is illustratively an interface which connects peripheral devices to the information processing apparatus 3.

The peripheral devices may include an input device which inputs information to the information processing apparatus 3, and an output device which outputs information generated by the information processing apparatus 3.

The input device may include a keyboard, a mouse and/or a touch panel. The output device may include a display and/or a printer.

By the way, when a heart rate is high immediately after an exercise of the user compared to a rest time, a breathing rate of the user is also high (in other words, a breathing activity of the user is active). Therefore, a motion of a breast accompanying the breathing activity tends to be great.

Hence, when the heart rate of the user is higher, a signal component (e.g. frequency component) corresponding to a motion accompanying the breathing activity of the user is more likely to be mixed in the detected signal of the wireless sensor 21 as a noise component with respect to the signal component (e.g. frequency component) corresponding to a motion accompanying the heartbeat of the user.

As a result, a probability that a breathing derived signal component (which may be abbreviated as a "breathing component") is erroneously detected as a heartbeat derived signal component (which may be referred to as a "heartbeat component") becomes high, and it becomes difficult to detect a heartbeat component from the detected signal of the wireless sensor 21. In other words, when the heart rate of the user is higher, the breathing component lowers a detection accuracy of heartbeat component.

For example, an average frequency band (which may be referred to as a "heartbeat appearance band" for descriptive purposes) in which a heartbeat component of the human body during a rest time is assumed to appear in the detected signal of the wireless sensor 21 is approximately 0.7 Hz to 4.0 Hz as a non-restrictive example.

By contrast with this, an average frequency band (which may be referred to as a "breathing appearance band" for descriptive purposes) in which a breathing component of the human body during a rest time is assumed to appear in the detected signal of the wireless sensor 21 is approximately 0.1 Hz to 0.5 Hz as a non-restrictive example.

Hence, in a state where the heartbeat and breathing of the user are stable as in a rest time, the heartbeat appearance band and the breathing appearance band tend not to overlap, and a probability that the breathing component is erroneously detected as a heartbeat component is low.

However, when the breathing activity of the human body becomes active, a harmonic (N-th harmonic) component which is N times (N is an integer equal to or more than 2) as a reference frequency corresponding to the breathing component appears in the heartbeat appearance band in some cases. When the harmonic component of the breathing component appears in the heartbeat appearance band, there is a probability that the harmonic component of the breathing component is erroneously detected as the heartbeat component.

As illustrated in, for example, FIG. 5, frequency analysis of the wireless sensor value causes a harmonic component such as a second harmonic to a fifth harmonic of the peak frequency to appear at a high frequency band side with respect to a primary peak frequency of the breathing component appearing in a breathing appearance band A of approximately 0.1 Hz to 0.5 Hz. In addition, a band in which a harmonic component of a breathing component appears will be referred to as a "harmonic appearance band" for descriptive purposes (see reference symbol C in FIG. 5). Further, an "N-th harmonic" may be referred to as an "N-th order harmonic component".

The peak frequency of the breathing component tends to shift more toward the high frequency side when the breathing rate of the observed person is higher. Therefore, a harmonic appearance band C may be also regarded to shift toward the high frequency side in response to the shift (see an dotted arrow C1 in FIG. 5).

When an upper limit frequency of the harmonic appearance band C shifts toward the high frequency side, at least part of the harmonic appearance band C at the high frequency side overlaps a low frequency side of a heartbeat appearance band B.

When at least part of the harmonic appearance band C overlaps the heartbeat appearance band B, a harmonic component of a breathing component is likely to appear in the heartbeat appearance band B.

The harmonic component of the breathing component becomes a noise component with respect to the heartbeat component. Therefore, the harmonic component of the breathing component is more likely to be erroneously detected as a heartbeat component.

Hence, in the present embodiment, for example, the filter suppresses a signal component of the harmonic appearance band C in the detected signal of the wireless sensor 21. The HPF may be illustratively applied to the filter.

FIG. 6 illustrates an example of the HPF characteristics. FIG. 6 illustrates the HPF characteristics whose three types of cut-off frequencies are 0.8 Hz, 1.0 Hz and 1.3 Hz as a non-restrictive example. The HPF characteristics applied to a wireless sensor value may be illustratively selected and determined from the three types of the HPF characteristics.

In addition, the harmonic appearance band C can be shifted in a frequency domain response to a change in the breathing rate as described above. Therefore, the HPF characteristics (e.g. cut-off frequency) may also be varied in response to the change in the breathing rate.

HPF characteristics variable control maybe illustratively performed in a frequency range which does not include the heartbeat appearance band B such that the heartbeat component in the heartbeat appearance band B is not cut. In other words, the HPF characteristics variable control may be performed such that the HPF cutoff band does not overlap the heartbeat appearance band B.

In this regard, when the heart rate of the observed person is higher, the breathing rate tends to be higher. Therefore, in response to a shift of a peak frequency of a breathing component toward the high frequency side, the heartbeat appearance band B may be also regarded to shift toward the high frequency side a (dotted arrow B1 in FIG. 5).

Hence, in response to the shift of the heartbeat appearance band B toward the high frequency side, it is possible to effectively suppress a harmonic component of a breathing component by setting such a high frequency range that upper limit frequency of the HPF cutoff band does not overlap the heartbeat appearance band B.

For example, one of the three types of the HPF characteristics illustrated in FIG. 6 may be adaptively selected according to the breathing rate of the observed person. As a non-restrictive example, when the breathing rate of the observed person is higher, higher HPF characteristics of the cut-off frequency may be selected.

In addition, the breathing rate of the observed person can be detected or estimated as a result of analyzing a frequency of the detected signal of the wireless sensor 21 or based on a detected signal of the inertial sensor 22 as described below.

By applying the above HPF to the detected signal of the wireless sensor 21, it is possible to effectively suppress a harmonic component of a breathing component appearing in the heartbeat appearance band B and, consequently, improve a detection accuracy of heartbeat signal. Since the detection accuracy of heartbeat signal, it is also possible to improve, for example, an accuracy and efficiency to control a spatial environment in which the observed person is positioned.

(Operation example)

Some operation examples of the sensor system 1 according to the present embodiment will be described. In addition, an example where a wireless sensor value of the vital sensor 2 is processed by the information processing apparatus 3 (e.g. processor 31) will be described below. In this regard, part or an entirety of the process may be performed by the vital sensor 2 (e.g. processor 23).

(First embodiment)

FIG. 7 is a flowchart illustrating an operation example of a sensor system 1 according to the first embodiment. As illustrated in FIG. 7, an information processing apparatus 3 receives a wireless sensor value from a sensor unit 2 (processes P11). The received wireless sensor value may be stored in a storage unit 30.

The information processing apparatus 3 may apply a HPF to a wireless sensor value (process P12). The wireless sensor value to which the HPF is applied may be a wireless sensor value read from the storage unit 30 or a wireless sensor value received in process P11.

HPF characteristics applied to the wireless sensor value may be set to characteristics having one of cut-off frequencies illustrated in FIG. 6. In addition, in the first embodiment, the HPF characteristics may be set statically. Dynamically setting the HPF characteristics will be described below in a second embodiment and a third embodiment.

Applying the HPF suppresses a signal component of a harmonic appearance band C of a breathing component in a wireless sensor value, so that a signal component of a heartbeat appearance band B passes through the HPF without being suppressed. The information processing apparatus 3 may analyze the frequency of the signal component having passed through the HPF (process P13) .

For frequency analysis, fast Fourier transform (FFT) or discrete Fourier transform (DFT) may be used. The wireless sensor value is converted from a time domain signal into a frequency domain signal (which may be referred to as a "frequency signal" for descriptive purposes) by FFT or DFT.

The information processing apparatus 3 may detect, for example, a frequency having a peak value (such a frequency may be referred to as a "peak frequency") in the heartbeat appearance band B of a frequency signal obtained by the frequency analysis. The peak frequency corresponds to a peak frequency of a heartbeat component.

By multiplying the peak frequency of the heartbeat component with, for example, 60 (second), it is possible to convert the frequency into a heart rate. The information processing apparatus 3 may determine BPF characteristics applied to a signal component having passed through the HPF, based on the peak frequency of the detected heartbeat component (or the heart rate) (process P14).

In this regard, as illustrated in FIG. 8, for example, a time length (or a time interval) including a heartbeat per beat tends to vary according to whether the heart rate per unit time is high or low. In addition, in FIG. 8, a horizontal axis indicates a heart rate x (n) (n is a positive integer) at a given point of time, and a vertical axis indicates a following heart rate x (n+1). Meanings of reference numeral 122, reference numeral 124, reference numeral 126 and "d" in FIG. 8 will be described below.

For example, as the heart rate per unit time increases, a variation of the time length per beat tends to decrease. Conversely, as the heart rate per unit time decreases, a variation of the time length per beat tends to decreases.

Illustratively, in the heartbeat appearance band B (0.7 to 4.0 Hz in a non-restrictive example), a variation of approximately ±20% occurs in a time length per beat in case of 50 beats per unit time (e.g. one minute). Meanwhile, a variation of approximately ±5% occurs in a time length per beat in case of 120 beats higher than the 50 beats. Such a "variation" may be regarded as "heartbeat characteristics" of the biological object.

Hence, when a passband width of a BPF is statically set according to a specific heart rate such as 60 beats or 120 beats, a heartbeat component lacks or a noise component cannot be sufficiently canceled according to whether the heart rate is high or low. As a result, a detection accuracy of the heart rate may be decreased.

Hence, in the present embodiment, the passband width of the BPF (which is also abbreviated as a "BPF bandwidth" below) may be adaptively changed according to whether a heart rate is high or low, in other words, whether a peak frequency of a heartbeat component is high or low.

When, for example, the heart rate per unit time is higher, the BPF bandwidth may be set to be narrower and, when the heart rate per unit time is lower, the BPF bandwidth may be set to be wider. In addition, a BPF bandwidth variable setting example will be described below with reference to FIGS. 19 to 28.

By making such a BPF bandwidth variable setting, it is possible to reduce a miss to receive a heartbeat component in the heartbeat appearance band B and enhance the degree of suppression of a noise component different from the heartbeat component.

Even when, for example, the heartbeat appearance band B includes a harmonic component of a breathing component which has not been able to be completely suppressed by a HPF, when the harmonic component is outside the BPF bandwidth, it is possible to suppress the harmonic component.

Further, even when the heartbeat appearance band B includes a noise component deriving from a motion accompanying an activity of the observed person, when the noise component is outside the BPF bandwidth, it is possible to suppress the noise component likewise.

In addition, whether the heart rate is high or low can be illustratively determined according to whether a peak frequency is high or low in a result obtained by analyzing the frequency of a wireless sensor value.

According to determination of the BPF characteristics (process P14 in FIG. 7), the information processing apparatus 3 may apply the BPF of the determined BPF characteristics to a HPF-applied frequency signal (process P15).

The information processing apparatus 3 may search for and detect a heartbeat component in the heartbeat appearance band B based on the BPF-applied frequency signal. For example, the information processing apparatus 3 may detect a heartbeat component indicative of a distinctive change corresponding to a heartbeat of the observed person as a "feature point" in the heartbeat appearance band B (process P16).

The "feature point" may be, for example, a point (in other words, a peak value) at which a first derivation becomes zero in a signal waveform of the BPF applied wireless sensor value. In response to detection of the "feature point", the information processing apparatus 3 can calculate a heart rate per minute by calculating a time interval (e.g. "second") at the "feature point" and dividing one minute (= 60 seconds) by the calculated time interval (process P17).

The calculated heart rate may be used as a parameter to control a spatial environment in which the observed person is positioned (process P18). Further, the information of the calculated heart rate may be optionally outputted to an output device such as a display or a printer as indicated by the dotted line in FIG. 7 (process P19).

In addition, at least one of the frequency analysis result in process P13, information of the BPF characteristics determined in process P14 and information of the feature point detected in process P16 may be optionally outputted to the output device such as the display or the printer. In this case, the frequency analysis result, and whether or not a setting status or a setting of the BPF characteristics is appropriate can be checked by using the output device.

As described above, according to the first embodiment, by applying to a wireless sensor value the HPF having a cutoff band which can suppress a breathing component and a harmonic component of the breathing component, it is possible to suppress in the wireless sensor value a breathing derived noise component which is likely to be mixed in the heartbeat appearance band B.

Consequently, it is possible to reduce a probability that the breathing derived noise component is erroneously detected as a heartbeat component in the heartbeat appearance band B and improve heartbeat component detection precision based on a wireless sensor value.

Even when a heart rate is high and a breathing rate is also high after an exercise of the observed person, for example, it is possible to suppress a breathing derived noise component in the heartbeat appearance band B. Consequently, even in an active state of a breathing activity of the observed person after the exercise, it is possible to improve precision to detect the heart rate of the observed person.

In addition, FIG. 9 illustrates an example of signal waveforms in a frequency domain of a wireless sensor value in case where the HPF (a cut-off frequency is 0.8 kHz) is applied to a wireless senor value and in case where the HPF is not applied to wireless sensor value.

FIG. 9 illustrates an example of the signal waveform in case where the HPF is not applied to the signal waveform indicated by a dotted line, and illustrates an example of the signal waveform in case where the HPF is applied to the signal waveform indicated by a solid line. In FIG. 9, the non-HPF-applied signal waveform indicated by the dotted line corresponds to the signal waveform illustrated in FIG. 5.

As illustrated in FIG. 9, a breathing component and harmonic components of a second harmonic to a fifth harmonic of the breathing component are suppressed in the HPF-applied signal waveform compared to the non-HPF-applied signal waveform.

In addition, the heartbeat appearance band B includes a noise component different from a breathing derived noise component in some cases. For example, the heartbeat appearance band B includes a noise component deriving from a motion accompanying an activity such as walking of the observed person.

Even when the heartbeat appearance band B includes a noise component different from a breathing derived noise component, it is possible to suppress the noise component together with the breathing derived noise component as long as the noise component is included in the HPF cutoff band.

(Second embodiment)

FIG. 10 is a flowchart illustrating an operation example of a sensor system 1 according to the second embodiment. An example where HPF characteristics are adaptively controlled according to a breathing rate of an observed person will be described in the second embodiment.

As illustrated in FIG. 10, when receiving a wireless sensor value from a vital sensor 2 (process P11), an information processing apparatus 3 may analyze the frequency of the received wireless sensor value by FFT or DFT (process P21). In addition, the received wireless sensor value may be stored in a storage unit 30.

The information processing apparatus 3 may search for and detect a peak frequency in a breathing appearance band A (approximately 0.1 Hz to 0.5 Hz as a non-restrictive example) in a frequency signal obtained by the frequency analysis. The detected peak frequency corresponds to a primary peak frequency of a breathing component.

The information processing apparatus 3 may determine HPF characteristics based on the peak frequency of the breathing component (process P22).

For example, the information processing apparatus 3 may determine the HPF characteristics such that each signal component in the breathing appearance band A including the peak frequency of the breathing component and in a harmonic appearance band C in which a harmonic component corresponding to an N-th harmonic compared to the peak frequency of the breathing component appears is suppressed.

In this regard, when, for example, a breathing rate of an observed person is higher, the peak frequency of the breathing component also becomes higher (in other words, the peak frequency shifts toward a high frequency side).

In response to the shift of the peak frequency of the breathing component toward the high frequency, the harmonic component of the peak frequency of the breathing component is likely to appear in a heartbeat appearance band B.

Consequently, the information processing apparatus 3 may adaptively control a cut-off frequency of the HPF applied to a wireless sensor value, according to whether the peak frequency of the breathing component is high or low. For example, the information processing apparatus 3 may perform control to set the cut-off frequency of the HPF applied to a wireless sensor value, to a higher frequency when the peak frequency of the breathing component is higher.

In an example in FIG. 6, when the peak frequency of the breathing component is higher, the information processing apparatus 3 may select the highest cut-off frequency among three types of cutoff frequencies of 0.8 Hz, 1.0 Hz and 1.3 Hz to determine as the HPF characteristics.

According to the determination of the HPF characteristics, the information processing apparatus 3 may apply the HPF of the HPF characteristics determined to the wireless sensor value (process P12). The wireless sensor value to which the HPF is applied may be read from the storage unit 30.

Subsequently, the information processing apparatus 3 may perform processes P13 to P19 similar to the first embodiment (e.g. FIG. 7).

For example, the information processing apparatus 3 may apply a BPF having a center frequency and a bandwidth matching the peak frequency of the heartbeat component, to the HPF-applied wireless sensor value, and calculate a heart rate based on the BPF-applied wireless sensor value. Information of the calculated heart rate may be used to control a spatial environment or may be outputted to an external device such as a display or a printer.

As described above, according to the second embodiment, the HPF characteristics applied to a wireless sensor are adaptively varied according to a peak frequency of a breathing component detected in a result obtained by analyzing a frequency of a wireless sensor value.

Consequently, it is possible to obtain the same function and effect as those of the first embodiment and strongly suppress breathing derived noise component in the heartbeat appearance band B compared to the first embodiment. Consequently, it is possible to further improve heartbeat component detection precision based on the wireless sensor value. The heartbeat component detection precision improves, so that precision to detect a heart rate of the observed person also improves.

(Third embodiment)

In the above-described second embodiment, an information processing apparatus 3 detects a peak frequency of a breathing component of an observed person based on a result obtained by analyzing a frequency of a wireless sensor value, and adaptively determines HPF characteristics according to the detected peak frequency.

An example where a vital sensor 2 includes an inertial sensor 22, and the information processing apparatus 3 adaptively determines HPF characteristics based on an inertial sensor value will be described in the third embodiment.

For example, the information processing apparatus 3 may estimate a heart rate of the observed person based on an inertial sensor value, estimate a breathing rate based on the estimated heart rate and adaptively determine HPF characteristics according to the estimated breathing rate.

FIG. 11 is a flowchart illustrating an operation example of a sensor system 1 according to the third embodiment. As illustrated in FIG. 11, the information processing apparatus 3 receives a wireless sensor value and an inertial sensor value from the vital sensor 2 (processes P11 and P31).

The information processing apparatus 3 may estimate a heart rate of the observed person based on the inertial sensor value in response to reception of the inertial sensor value (process P32). FIGS. 12 and 13 illustrate a first example of a heart rate estimation process, and FIGS. 14 to 16 illustrate a second example of the heart rate estimation process.

(First example of heart rate estimation process)

As illustrated in FIG. 12, the information processing apparatus 3 may calculate the number of steps (which may be referred to as a "walking cadence") of a user per unit time (illustratively one second) based on the inertial sensor value (process P321). Similar to general pedometers, the number of steps can be obtained by, for example, counting in, for example, a processor 31 the number of times that the inertial sensor value exceeds a given threshold value.

Here, the walking cadence and the heart rate can be linked by a relational equation. The relational equation may be illustratively derived by performing a curve-fitting on a plurality of actual measured values, for example.

A non-restrictive example of the relational equation of the walking cadence and the heart rate can be expressed by following polynominal equation (1) as illustrated in FIG. 13.

$$y=8E-05x^3-0.0011x^2-0.0855x+61.597\ldots(1)$$

In FIG. 13, a horizontal axis "x" indicates a walking cadence [the number of steps/second], and a vertical axis "y" indicates a heart rate per unit time (for example, one minute) [beats per minute, bpm]. Further, "8E-05" represents "$8x10^{-5}$".

The information processing apparatus 3 may calculate the heart rate by calculating equation (1) based on the walking cadence calculated in process P321 (process P324 in FIG. 12). The calculated heart rate may be referred to as an "estimated heart rate" for descriptive purposes.

The relation illustrated in FIG. 13 may be expressed by data in a table format (which may be referred to as "table data"), for example. The table data may be stored in a storage unit 30 (see FIG. 4) of the information processing apparatus 3, for example. The information processing apparatus 3 (e.g. processor 31) may find the estimated heart rate with respect to the walking cadence by referring to the table data without performing an arithmetic operation.

(Second aspect of heart rate estimation process)

Next, a second aspect of the heart rate estimation process (P32) in FIG. 11 will be described with reference to FIGS. 14 to 16. The above first example is an example where a heart rate is calculated based on a walking cadence of the observed person. In the second example, the heart rate may be calculated based on an exercise intensity of the observed person.

As illustrated in FIG. 14, similar to the first example (FIG. 12), the information processing apparatus 3 may calculate the walking cadence of the user based on the wireless sensor value (process P321).

The information processing apparatus 3 may calculate a walking speed based on the walking cadence according to the calculated walking cadence (process P322).

In this regard, the walking cadence and the walking speed can be linked by a relational equation. The relational equation may be derived by performing a curve-fitting on a plurality of actual measured values.

A non-restrictive example of the relational equation of the walking cadence and the walking speed can be expressed by following polynominal equation (2) as illustrated in FIG. 15.

$$y=1E-05x^3-0.0014x^2+0.0725x-0.0119...(2)$$

In FIG. 15, a horizontal axis "x" indicates a walking cadence [the number of steps/second], and a vertical axis "y" indicates a walking speed [km/h] per unit time (illustratively, one hour). Further, "1E-05" represents "$1x10^{-5}$".

As a walking distance per unit time increases, a step width of a distance per step and the number of steps also tend to increase. For example, a step width = height x 0.37 is applicable when the user walks 70 m per minute (= 4.2 km per hour), a step width = height x 0.45 is applicable when the user walks 90 m per minute (= 5.4 km per hour) and a step width = height x 0.5 is applicable when the user walks 110 m per minute (= 6.6 km per hour). Therefore, it is possible to approximately calculate the walking speed corresponding to a walking distance per unit time based on the above step width and walking cadence.

The information processing apparatus 3 may calculate the walking speed by calculating equation (2) based on the walking cadence calculated in process P321.

In addition, the relation illustrated in FIG. 15 is expressed by, for example, table data similar to the relation illustrated in FIG. 13, and be stored in the storage unit 30 (see FIG. 4). The information processing apparatus 3 may find the walking speed with respect to the walking cadence by referring to the table data without performing an arithmetic operation.

The information processing apparatus 3 may calculate an exercise intensity of the user based on the walking speed according to the calculated walking speed (process P323 in FIG. 14). The exercise intensity is an index value indicative of an activity amount of a person, and may be expressed by a METs value.

METs is an abbreviation of "Metabolic equivalents". The METs value may be a numerical value which expresses a relative value (e.g. a multiple number) of a metabolic rate (or a calorie consumption amount) during an activity of a person with respect to a metabolic rate during rest. A table in which METs values are associated with each activity of a person is referred to as a "METs table". The METs table is published by the National Institute of Health and Nutrition, for example.

FIG. 16 illustrates an example of a relation between walking speeds and METs values. A walking speed = 0 [km/h] is associated with a METs value = 1 and corresponds to a reference exercise intensity during rest. As illustrated in FIG. 11, as the walking speed increases, the METs value also increases.

For example, in case of a walking speed = 2.5 [km/h], the METs value is three times as a reference METs value (=1). In case of a walking speed = 4 [km/h], the METs value is five times as the reference METs value.

When the METs value is determined, a current heart rate can be determined based on an age of the user and the heart rate during rest, for example. The METs value can be expressed by following equation (3), for example.

$$\text{METs value} = (\text{heart rate} - \text{heart rate during rest}) / (\text{maximum heart rate} - \text{heart rate during rest}) \times 10 \ldots (3)$$

The "maximum heart rate" in equation (3) can be briefly calculated as "220 - age".

Consequently, the information processing apparatus 3 can calculate the current heart rate based on the METs value according to equation (3) (process P324 in FIG. 14). In other words, when it is difficult to precisely detect the heart rate during an exercise or an activity of the user, it is possible to estimate the heart rate based on the METs value according to equation (3) .

In this regard, the HPF does not need to be applied to a wireless sensor value depending on an estimated heart rate. For example, when the heart rate (or METs value) estimated based on the inertial sensor value is higher, the breathing rate also tends to be higher.

The information processing apparatus 3 estimates a breathing rate based on the estimated heart rate (process P35) when the estimated heart rate (or METs value) exceeds a threshold value in FIG. 11 (YES in process P33), and determines HPF characteristics applied to a wireless sensor value according to the estimated breathing rate (process P36).

When the estimated heart rate is higher, the higher breathing rate is estimated, and, when the breathing rate is higher, a harmonic component of a breathing component is more likely to be mixed in a heartbeat appearance band B. Therefore, similar to the first and second embodiments, application of the HPF to a wireless sensor value is determined.

Estimating the breathing rate based on the estimated heart rate may be performed based on relations between the heart rate and the breathing rate illustrated in FIGS. 17 and 18. In addition, FIG. 17 illustrates the relation between the heart rate and the breathing rate associated with a walking speed (which may be a METs value which is an index of an exercise intensity).

By, for example, performing curve-fitting (in other words, fitting of curves) by using a plurality of items of data of heart rates and breathing rates illustrated in FIG. 17, it is possible to derive a relational equation of the breathing rate and the heart rate illustrated in FIG. 18.

The relational equation can be illustratively expressed as in following polynomial (4).

$$y=0.0415x^2+0.8894x+58.035 \ldots (4)$$

In addition, a horizontal axis "x" in FIG. 18 indicates a breathing rate [f/min] per unit time (illustratively, one minute), and a vertical axis "y" in FIG. 18 indicates a heart rate [beats per minute, bpm] per unit time (illustratively, one minute).

The information processing apparatus 3 can calculate the breathing rate "x" by solving "x" in equation (4) by substituting the estimated heart rate in "y" in equation (4). The calculated breathing rate may be referred to as an "estimated breathing rate" for descriptive purposes.

In addition, the relation illustrated in FIG. 18 may be expressed by, for example, data of a table format (which may be referred to as "table data"). The table data may be stored in a storage unit 30 (see FIG. 4) of the information processing apparatus 3, for example. The information processing apparatus 3 (e.g. processor 31) may find the estimated breathing rate with respect to the estimated heart rate by referring to the table data without performing an arithmetic operation.

Further, the vertical axis (heart rate) in FIG. 18 may be replaced with a walking speed (or METs value). In other words, the heart rate tends to become higher when the walking speed (or METs value) is higher as illustrated in FIG. 17 (in other words, such a tendency means a correlation). Therefore, the breathing rate may be estimated based on a relation with the walking speed (or METs value).

When the estimated breathing rate is calculated, the information processing apparatus 3 may control a cut-off frequency of the HPF applied to a wireless sensor value, according to the estimated breathing rate similar to the second embodiment.

In an example in FIG. 6, when the peak frequency of the breathing component is higher, the information processing apparatus 3 may select the highest cut-off frequency among three types of cutoff frequencies of 0.8 Hz, 1.0 Hz and 1.3 Hz to determine as the HPF characteristics.

According to the determination of the HPF characteristics, the information processing apparatus 3 may apply the HPF of the determined HPF characteristics to the wireless sensor value (process P12 in FIG. 11). The wireless sensor value to which the HPF is applied may be read from the storage unit 30.

Subsequently, the information processing apparatus 3 may perform processes P13 to P19 in FIG. 11 similar to the first embodiment (e.g. FIG. 7).

For example, the information processing apparatus 3 may apply a BPF having a center frequency and a bandwidth matching the peak frequency of the heartbeat component, to the HPF-applied wireless sensor value, and calculate a heart rate based on the BPF-applied wireless sensor value. Information of the calculated heart rate may be used to control a spatial environment or may be outputted to an external device such as a display or a printer.

Meanwhile, when the heart rate (or METs value) estimated based on an inertial sensor value is a threshold value or less (NO) in process P33 in FIG. 11, the information processing apparatus 3 can sufficiently suppress a noise component appearing in the heartbeat appearance band B only by applying a BPF without applying a HPF to a wireless sensor value.

In other words, when the heart rate (or the METs value) estimated based on the inertial sensor value is less than the threshold value, an influence on detection of a heartbeat component caused by a harmonic component of a breathing component may be regarded to be little in some cases.

Consequently, when the estimated heart rate (or METs value) is the threshold value or less (NO in process P33), the information processing apparatus 3 may determine BPF characteristics applied to the wireless sensor value based on the estimated heart rate (process P34).

For example, the information processing apparatus 3 may convert the estimated heart rate into a frequency, and sets the converted frequency to a center frequency of the BPF applied to the wireless sensor value. In addition, the heart rate can be illustratively converted into the frequency by dividing the heart rate by 60 (seconds). Further, the information processing apparatus 3 may set a narrower bandwidth to the BPF when the estimated heart rate ishigher. A detailed determination example of the BPF characteristics will be described below.

The information processing apparatus 3 may apply the BPF of the determined BPF characteristics to the wireless sensor value, and filter the wireless sensor value (process P15). The wireless sensor value to which the BPF is applied in process P15 may be read from the storage unit 30.

Subsequently, the information processing apparatus 3 may perform processes P16 to P19 in FIG. 11 similar to the first embodiment (e.g. FIG. 7).

As described above, according to the third embodiment, a heart rate of the observed person is estimated based on an inertial sensor value, a breathing rate is estimated based on the estimated heart rate, and HPF characteristics applied to a wireless sensor value are adaptively determined according to the estimated breathing rate.

The inertial sensor value includes information indicating a "motion" corresponding to an activity of the observed person. Consequently, it is possible to estimate a heart rate associated with a "motion", and, when the heart rate can be estimated, it is possible to estimate a breathing rate. To sum up, it is possible to estimate a breathing rate of the observed person who is doing an activity, based on the inertial sensor value.

When the observed person is doing an activity, even when a frequency of a wireless sensor value is analyzed as in the second embodiment, a noise component derived from a motion accompanying the activity of the observed person lowers breathing component detection precision or makes it impossible to detect a breathing component depending on cases.

However, according to the third embodiment, it is possible to estimate a breathing rate based on an inertial sensor value, so that it is possible to determine HPF characteristics applied to a wireless sensor value as appropriate characteristics without analyzing a frequency of the wireless sensor value.

Consequently, even when the observed person is doing an activity, it is possible to make the HPF characteristics appropriate and suppress a breathing derived noise component in the heartbeat appearance band B. Consequently, even when the observed person is doing an activity, it is possible to improve precision to detect a heartbeat component of the observed person. The heartbeat component detection precision improves, so that precision to detect a heart rate of the observed person also improves.

Further, according to the third embodiment, when it is possible to determine that a heart rate (or METs value) estimated based on an inertial sensor value is the threshold value or less and therefore a breathing derived noise component hardly appears in the heartbeat appearance band B, the HPF does not need to be applied to a wireless sensor value

Consequently, compared to a case where HPF characteristics are adaptively determined uniformly irrespectively of whether the heart rate or the METs value estimated based on the inertial sensor value is high or low, it is possible to reduce a processing amount, in other words, a processing load of the information processing apparatus 3.

In addition, in the above third embodiment, a breathing rate of the observed person is estimated based on a relation between an inertial sensor value and a heart rate (or METs value). However, the breathing rate may be estimated based on a relation with the inertial sensor value. When, for example, an average value of inertial sensor values per given unit time is higher, a motion amount of the observed person is larger. Therefore, whether a breathing rate is high or low may be estimated according to whether the inertial sensor value is high or low.

(Determination example of BPF characteristics)

Next, an example of a process (e.g. process P14 in FIGS. 7, 10 and 11 and process P34 in FIG. 11) of determining (or setting) the above-described BPF characteristics will be described with reference to FIGS. 8 and 19 to 28.

A determination example of the BPF characteristics described below may be common between the first and third embodiments. In this regard, in the following description, a "reference heart rate" in process P14 in FIGS. 7, 10 and 11 corresponds to a heart rate which is detected based on a frequency analysis result obtained after applying a HPF to a wireless sensor value and which is associated with a peak frequency of a heartbeat component.

By contrast with this, the "reference heart rate " in process P34 in FIG. 11 corresponds to a heart rate estimated based on an inertial sensor value. For example, in the third embodiment, the "reference heart rate" in case where NO is determined in threshold determination process P33 in FIG. 11 corresponds to the heart rate estimated based on the inertial sensor value in process P32.

As described above, FIG. 8 illustrates a relation between a heart rate per unit time and a variation of a time length per heartbeat. Reference numeral 122 denotes measured data (x(n), x(n+1)) of the heart rate and a straight line 124 indicates a median value of each measured data 122.

The straight line indicating the median value 124 is illustratively a straight line of each measured data 122 calculated according to a least-squares method, for example, and is assumed to be expressed as ax (n) + bx(n+1) + c = 0. In this regard, coefficients a, b and c are actual numbers.

Here, a length d of a perpendicular line drawn from each measured data 122(x(n), x(n+1)) to the straight line 124 can be expressed by following equation (5).

$$\mathrm{d} = \frac{|ax(n)+bx(n+1)+c|}{\sqrt{a^2+b^2}} \quad \ldots (5)$$

A measured point having a maximum value of the length d is assumed to be a maximum distance point 126 expressed by a coordinate (x(m), x(m+1)). The "m" represents an positive integer which satisfies m ≤ n.

A straight line which passes through the maximum distance point 126 and a coordinate (a coordinate (120,114) in the example in FIG. 8) corresponding to "-0.1 Hz" in case of heartbeat frequency = 2 Hz represents a lower limit of a bandwidth, for example.

The straight line indicative of an upper limit of the bandwidth corresponds to a straight line which passes through a point (120, 126) and a point having the same width as that of the lower limit of a bandwidth at any of frequencies, for example. A bandwidth in case of heartbeat frequency = 2 Hz may be determined with reference to a maximum value and a minimum value of a next heartbeat at heart rate x(n) = 120 of the measured data 122 in FIG. 8.

Next, FIG. 19 is a diagram illustrating a setting example of the BPF characteristics. FIG. 19 illustrates an example of an upper limit and a lower limit of a band of the BPF calculated by substituting the reference heart rate in a preset equation.

In FIG. 19, a horizontal axis indicates a reference heart rate [Hz], and a vertical axis indicates a heart rate [Hz] corresponding to an upper limit and a lower limit of a bandwidth. In FIG. 19, the upper limit of the bandwidth is indicated by a band upper limit value 132, and the lower limit of the bandwidth is indicated by a band lower limit value 134. FIG. 19 illustrates the upper limit values and the lower limit values of the bandwidth at some reference heart rate.

The above "preset equation" may be determined by interpolation or extrapolation based on, for example, the maximum distance point 126 and reference coordinates (120, 126) and (120, 114) determined based on the measured data 122 illustrated in FIG. 8.

Next, FIG. 20 is a diagram illustrating an example of a passband of the BPF. In FIG. 20, a horizontal axis indicates a center frequency of the BPF, and a vertical axis indicates a bandwidth of the BPF. Further, in FIG. 20, a fixed minimum heartbeat 141 or a variable minimum heartbeat 144 indicates a heart rate corresponding to a lower limit of a band when a bandwidth corresponding to a given center frequency is adopted.

Furthermore, in FIG. 20, a fixed maximum heartbeat 142 or a variable maximum heartbeat 145 indicates a heart rate corresponding to a lower limit of a band when a bandwidth corresponding to a given center frequency is adopted.

The fixed minimum heartbeat 141 and the fixed maximum heartbeat 142 are comparative examples in case where a bandwidth does not change according to a heart rate in comparison with a case where the bandwidth of the BPF is variable in the present embodiment.

According to the variable minimum heartbeat 144 and the variable maximum heartbeat 145, the bandwidth of the BPF corresponds to a bandwidth indicated by reference numeral 148 near heart rate = 1 Hz and corresponds to a bandwidth indicated by reference numeral 149 which is narrower than the bandwidth 148 near heart rate = 4 Hz, for example. Thus, the wider bandwidth 148 is set at a lower heart rate, and the narrower bandwidth 149 is set at a higher heart rate.

FIG. 21 is a diagram illustrating a setting example of the BPF bandwidth. In FIG. 21, a horizontal axis indicates a center frequency, and a vertical axis indicates a bandwidth. In FIG. 21, a straight line 152 indicates an example of a setting value of a bandwidth with respect to the center frequency. For example, in examples described with reference to FIGS. 8 and 20, the bandwidth 152 may be set to decrease linearly in response to an increase of the center frequency.

A relation between the center frequency and the bandwidth may be enough to have a relation in which the bandwidth becomes wider when the center frequency becomes lower, and does not need to be a relation expressed by a straight line. For example, as indicated by reference numeral 154 in FIG. 21, the bandwidth may be set such that the bandwidth changes stepwise with respect to the center frequency. The relation between the bandwidth and the center frequency may be determined by interpolation and extrapolation using one or more suitable functions of a monotonic increase based on given two or more points determined based on an actual measured value, for example.

Next, FIGS. 22 to 25 are diagrams illustrating other setting examples of bandwidths. FIGS. 22 and 23 are diagrams illustrating examples of a heart rate distribution, and FIGS. 24 and 25 are diagrams illustrating examples of a heart rate statistical process. In FIGS. 22 and 23, horizontal axes indicate a time indicative of a difference between a heartbeat interval at a given point of time and a next heartbeat interval, and vertical axes indicate the number of measured times.

FIG. 22 illustrates a heart rate distribution whose heart rate is near 55 to 60, and FIG. 23 illustrates a heart rate distribution whose heart rate is near 75 to 80. In comparison between FIGS. 22 and 23, items of measured data concentrate more near the vertical axis at the heart rate near 55 to 60 than at the heart rate near 75 to 80, and the heart rate varies less. The measured data used for a statistical process may be data measured by an electrocardiograph which measures a heartbeat by placing an electrode in contact with a biological object, for example.

FIGS. 24 and 25 illustrate examples of the statistical process of actual measured data at the heart rate near 60 and at the heart rate near 120. In examples of the statistical process illustrated in FIGS. 24 and 25, a probability distribution of 532 items of data is calculated, and the bandwidth of the BPF is calculated such that the measured data is in a range of a certain probability or more.

In the example of the statistical process in FIG. 24, by determining a range of 8 $\sigma$ of the calculated probability distribution and CP value = 1.33 at the heart rate near 60, the bandwidth is $\pm$0.165 Hz. Here, $\sigma$ represents a standard deviation, and "CP" represents "Process Capability". The same bandwidth as this bandwidth at the heart rate near 120 is provided in case of CP value = 2.16.

Similarly, in the example of the statistical process in FIG. 25, by determining a range of 8$\sigma$ of the calculated probability distribution and CP value = 1.33 at the heart rate near 120, the bandwidth is $\pm$0.1015 Hz. The same bandwidth as this bandwidth at the heart rate near 60 is provided in case of CP value = 0.82.

The statistical process of an actual measured value is performed as described above, and the bandwidth of the BPF which enables data detection at a given probability or more is set. In this case, the relation between the bandwidth and the center frequency corresponding to the reference heart rate may be linearly determined based on given two points or may be determined by interpolation by performing the above statistical process on the center frequency of a shorter interval.

Alternatively, the relation between the bandwidth and the center frequency may be determined by interpolation and extrapolation by using one or more suitable functions of a monotonic increase based on each point when the given two or more points are determined, or may be set such that the bandwidth changes stepwise with respect to the center frequency.

FIGS. 26 and 27 are diagrams illustrating setting examples of a bandwidth. In FIGS. 26 and 27, horizontal axes indicate a frequency [Hz], and vertical axes indicate a gain [dB]. In FIGS. 26 and 27, a heartbeat appearance band 162 is 0.8 to 4.0 Hz, for example.

According to a frequency analysis result (e.g. FFT result) 160 of a wireless sensor value illustrated in FIG. 26, a peak gain indicated by reference numeral 163 exists in the heartbeat appearance band 162. A frequency corresponding to the peak gain 163 is set to the center frequency of the BPF, and a bandwidth indicated by reference numeral 164 is set to the bandwidth of the BPF, for example.

Meanwhile, according to an FFT result 165 illustrated in FIG. 27, a peak gain indicated by reference numeral 168 exists in the heartbeat appearance band 162. The frequency corresponding to the peak gain 168 is set to the center frequency of the BPF.

Here, since the center frequency corresponding to the peak gain 168 is higher than the frequency corresponding to the peak gain 163 in FIG. 26, a bandwidth 166 narrower than the bandwidth 164 in FIG. 26 is set to the bandwidth of the BPF.

Thus, the center frequency of the BPF is set to the frequency associated with the reference heart rate, and the bandwidth is subjected to adaptive variable control according to whether the center frequency is high or low. Consequently, it is possible to efficiently suppress a noise component appearing in the heartbeat appearance band.

In other words, by using the BPF which has the variable center frequency and bandwidth matching heartbeat characteristics of a biological object, it is possible to efficiently suppress an unnecessary signal component in the heartbeat appearance band without depending on whether the heart rate is high or low. Consequently, it is possible to improve heartbeat signal detection precision in the heartbeat appearance band.

FIG. 28 is a view illustrating an example of bandwidth information. Bandwidth information 170 illustrated in FIG. 28 may be stored in the storage unit 30 of the information processing apparatus 3, for example. As a non-restrictive example, the bandwidth information 170 may include reference heart rate information 171, BPF width lower limit information 172 and BPF width upper limit information 173.

The processor 31 of the information processing apparatus 3 is available to determine and set a band of the BPF corresponding to the reference heart rate with reference to the bandwidth information 170.

(Others)

In addition, application of a BPF to a wireless sensor value and BPF characteristics variable control may be optional in each of the above embodiments. It is expected that applying at least the above HPF to a wireless sensor value improves heartbeat component detection precision without applying the BPF.

Further, an example where a wireless sensor 21 and an inertial sensor 22 are integrated in a sensor unit 2 has been described in the third embodiment. However, the wireless sensor 21 and the inertial sensor 22 may be separate bodies as long as the wireless sensor 21 and the inertial sensor 22 are attached to the same user. In other words, whether the wireless sensor 21 and the inertial sensor 22 are integrated or are separate bodies does not matter as long as the same user is a sensing target.

When the wireless sensor 21 and the inertial sensor 22 are integrated as the sensor unit 2, it is possible to omit a labor to individually manage the wireless sensor 21 and the inertial sensor 22 or attach the wireless sensor 21 and the inertial sensor 22 to the user, and improve user-friendliness and convenience. Further, it is possible to prevent or suppress that attachment of one of the wireless sensor 21 and the inertial sensor 22 is forgotten or one of the wireless sensor 21 and the inertial sensor 22 is lost.

Meanwhile, when the wireless sensor 21 and the inertial sensor 22 are the separate bodies, it is possible to individually adjust attachment positions of the respective sensors 21 and 22 with respect to the user and expect improvement in the degree of freedom of the attachment positions.

## Claims

**1.** A sensor information processing apparatus comprising:

a receiver (34) configured to receive a detected signal of a wireless sensor (21); and
a processor (31) configured to:

apply to the detected signal a filter configured to cut at least a harmonic component of a frequency component derived from breathing of a person to be observed; and
detect a heartbeat component of the person in a signal having passed through the filter.

**2.** The sensor information processing apparatus according to claim 1, wherein the processor (31) detects a peak frequency having a peak value in a frequency band lower than a frequency band in which the heartbeat component is assumed to appear in a result obtained by analyzing a frequency of the detected signal of the wireless sensor (21), and
sets to a cutoff band of the filter a frequency band including the peak frequency and a frequency corresponding to a N-th harmonic (N is an integer equal to or more than two) of the peak frequency.

**3.** The sensor information processing apparatus according to claim 1 or 2, wherein the processor (31) sets the cutoff band of the filter to a band not overlapping with the frequency band in which the heartbeat component is assumed to appear in the detected signal.

**4.** The sensor information processing apparatus according to claim 2 or 3, wherein
the filter is a high-pass filter, and
the processor (31) sets a higher cutoff frequency of the high-pass filter when the peak frequency is higher.

**5.** A sensor information processing apparatus comprising:

a receiver (34) configured to receive a detected signal of a wireless sensor (21) and a detected signal of an inertial sensor (22); and
a processor configured to:

control a cutoff frequency of a high-pass filter applied to the detected signal of the wireless sensor (21), according to the detected signal of the inertial sensor (22); and
detect a heartbeat component of a person to be observed in a signal having passed through the high-pass filter.

**6.** The sensor information processing apparatus according to claim 5, wherein the processor (31) estimates a breathing rate of the observed person based on the detected signal of the inertial sensor (22), and sets the cutoff frequency of the high-pass filter to a frequency according to the estimated breathing rate.

**7.** The sensor information processing apparatus according to claim 6, wherein the processor (31) estimates a heart rate or an exercise intensity of the person based on the detected signal of the inertial sensor (22), and estimates the breathing rate based on the estimated heart rate or exercise intensity.

**8.** The sensor information processing apparatus according to claim 6 or 7, wherein the processor (31) sets a higher cutoff frequency of the high-pass filter when the estimated breathing rate is higher.

**9.** The sensor information processing apparatus according to any one of claims 5 to 8, wherein at least a harmonic component of a frequency component derived from breathing of the person is included in a cutoff band of the high-pass filter corresponding to the cutoff frequency.

**10.** The sensor information processing apparatus according to claim 9, wherein the processor (31) sets the cutoff band to a band not overlapping with the frequency band of the detected signal of the wireless sensor (21) in which the heartbeat component is assumed to appear.

**11.** The sensor information processing apparatus according to claim 7, wherein the processor (31) detects the heartbeat component in the detected signal of the wireless sensor (21) without applying the high-pass filter, when the estimated heart rate or exercise intensity is equal to or less than a threshold value.

**12.** The sensor information processing apparatus according to any one of claims 5 to 11, wherein the wireless sensor (21) and the inertial sensor (22) are provided in a sensor unit (2).

**13.** A sensor unit (2) comprising:

a wireless sensor (21); and
a processor (23) configured to:

apply to a detected signal of the wireless sensor (21) a filter configured to cut at least a harmonic component of a frequency component derived from breathing of a person to be observed; and
detect a heartbeat component of the person in a signal having passed through the filter.

**14.** A sensor unit (2) comprising:

a wireless sensor (21);
an inertial sensor (22); and
a processor (23) configured to:

control a cutoff frequency of a high-pass filter applied to the detected signal of the wireless sensor (21), according to the detected signal of the inertial sensor; and
detect a heartbeat component of a person to be observed in a signal having passed through the high-pass filter.

**15.** A sensor information processing program for causing a computer to execute a process comprising:

applying to a detected signal of a wireless sensor (21) a filter configured to cut at least a harmonic component of a frequency component derived from breathing of a person to be observed; and
detecting a heartbeat component of the person in a signal having passed through the filter.

**16.** A sensor information processing program for causing a computer to execute a process comprising:

controlling a cutoff frequency of a high-pass filter applied to a detected signal of a wireless sensor (21), according to a detected signal of an inertial sensor (22); and
detecting a heartbeat component of a person to be observed in a signal having passed through the high-pass filter.

FIG. 1

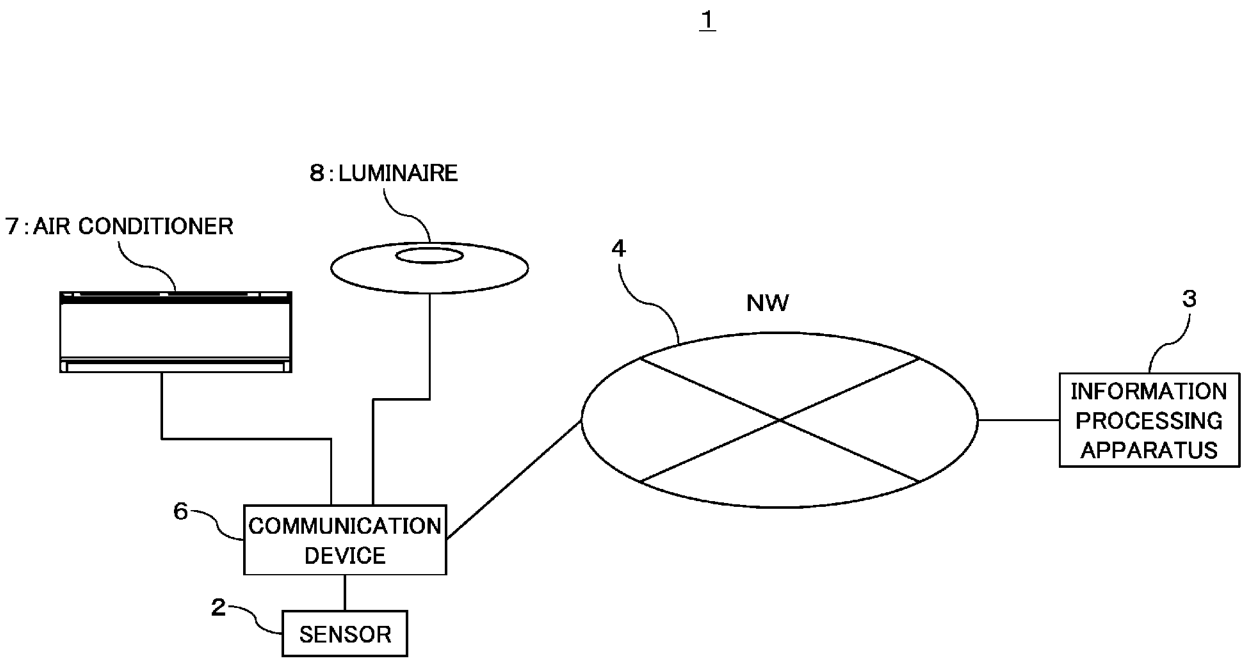
1
8 : LUMINAIRE
7 : AIR CONDITIONER
4
NW
3
INFORMATION PROCESSING APPARATUS
6
COMMUNICATION DEVICE
2
SENSOR

FIG. 2

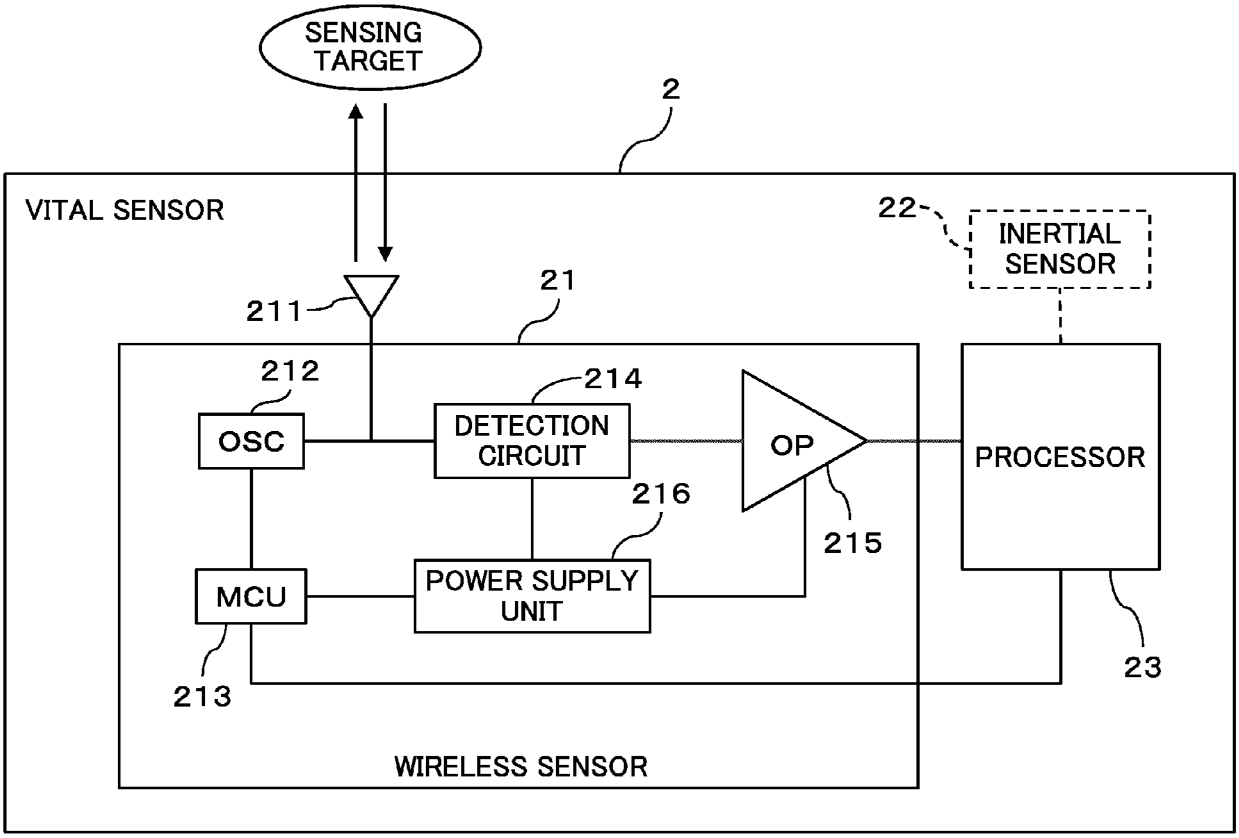
SENSING TARGET
2
VITAL SENSOR
22
INERTIAL SENSOR
21
211
212
214
OSC
DETECTION CIRCUIT
OP
PROCESSOR
216
215
MCU
POWER SUPPLY UNIT
213
23
WIRELESS SENSOR

FIG. 3

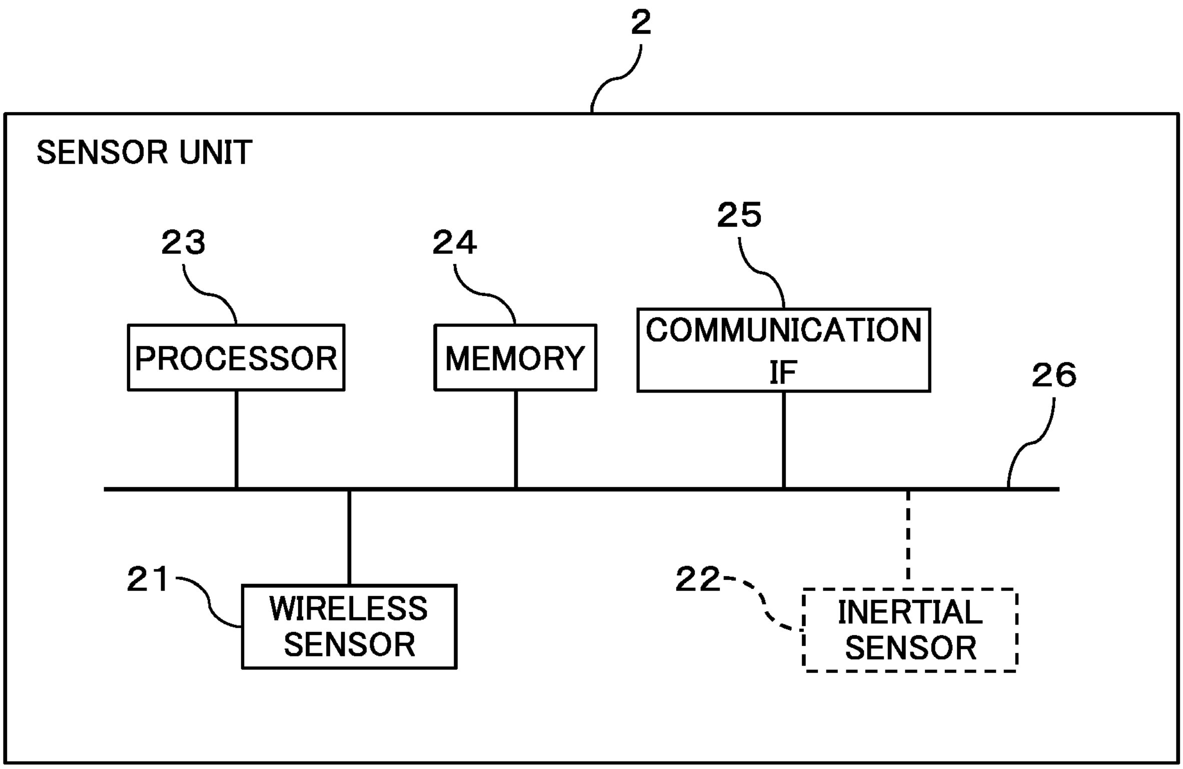
2
SENSOR UNIT
23
PROCESSOR
24
MEMORY
25
COMMUNICATION IF
26
21
WIRELESS SENSOR
22
INERTIAL SENSOR

FIG. 4

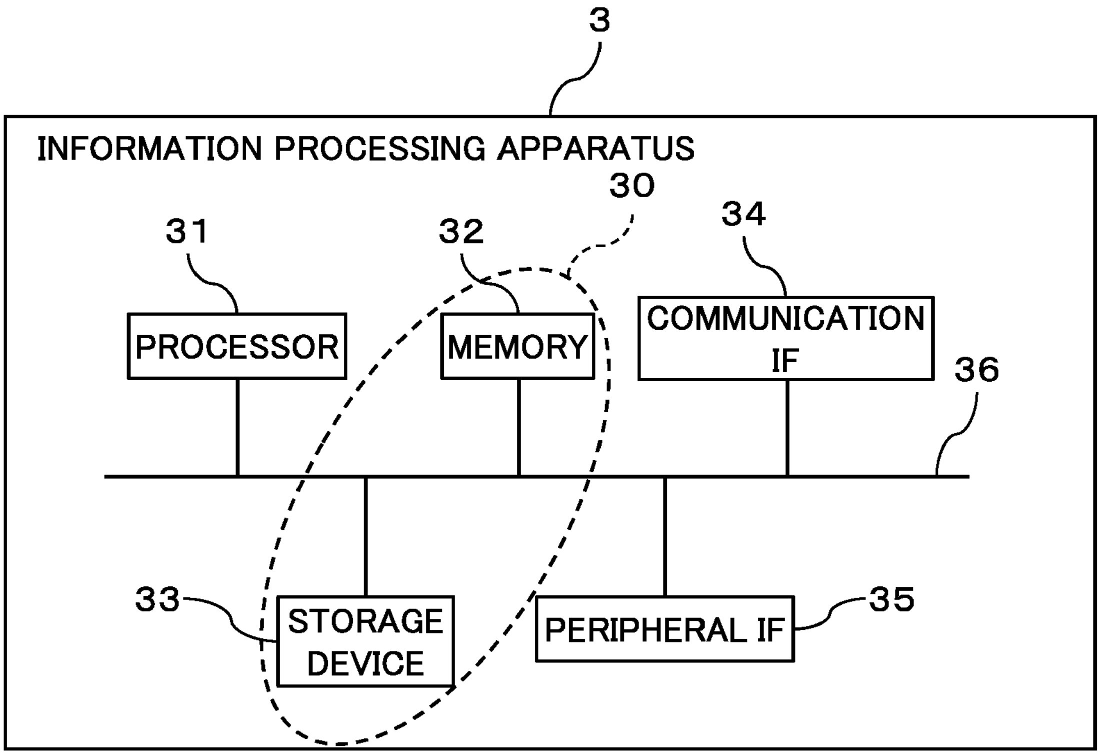
3
INFORMATION PROCESSING APPARATUS
30
31
32
34
PROCESSOR
MEMORY
COMMUNICATION IF
36
33
STORAGE DEVICE
PERIPHERAL IF
35

FIG. 5

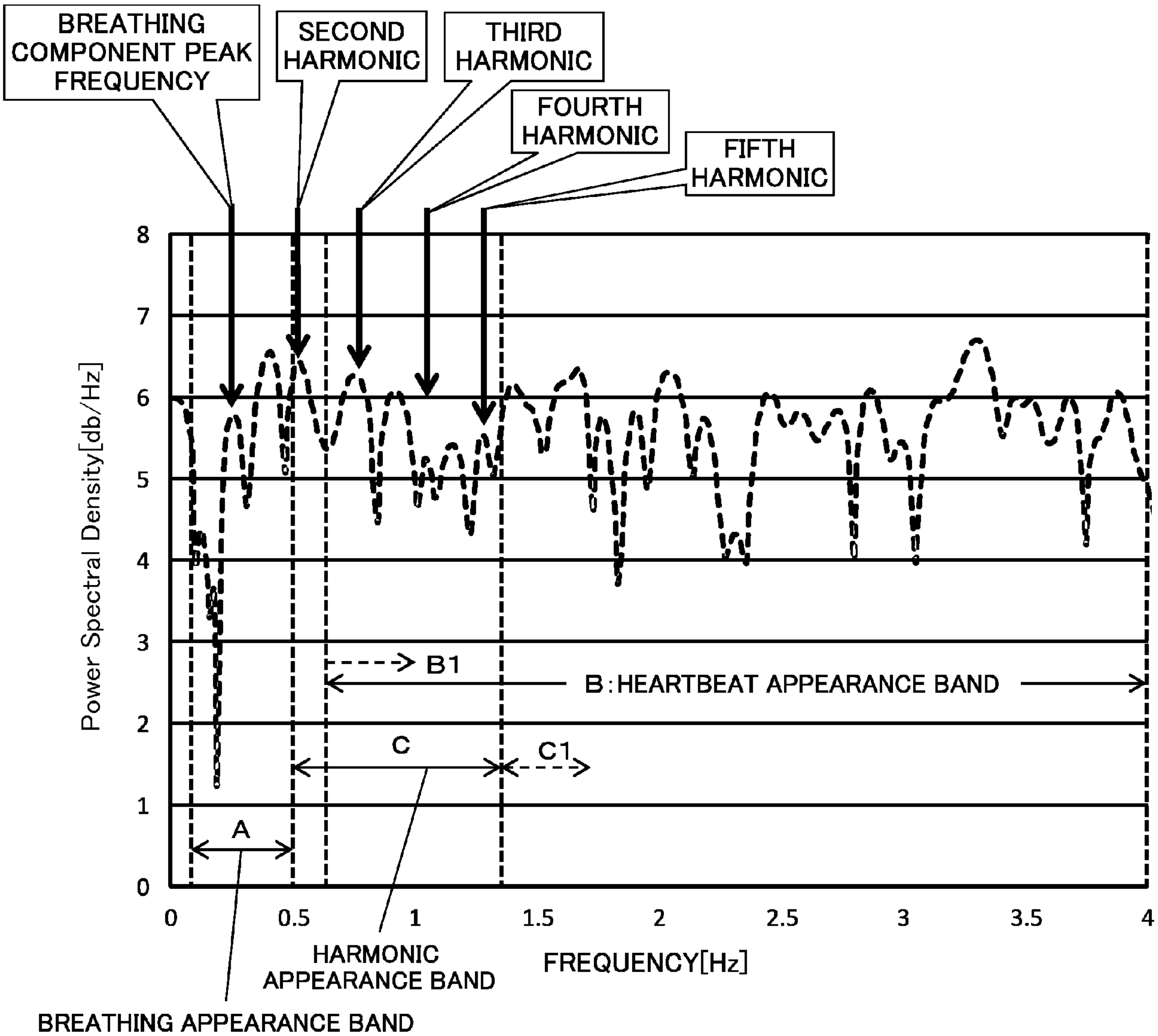
BREATHING COMPONENT PEAK FREQUENCY
SECOND HARMONIC
THIRD HARMONIC
FOURTH HARMONIC
FIFTH HARMONIC
Power Spectral Density[db/Hz]
8
7
6
5
4
3
2
1
0
B1
B：HEARTBEAT APPEARANCE BAND
C
C1
A
0
0.5
1
1.5
2
2.5
3
3.5
4
HARMONIC APPEARANCE BAND
FREQUENCY[Hz]
BREATHING APPEARANCE BAND

FIG. 6

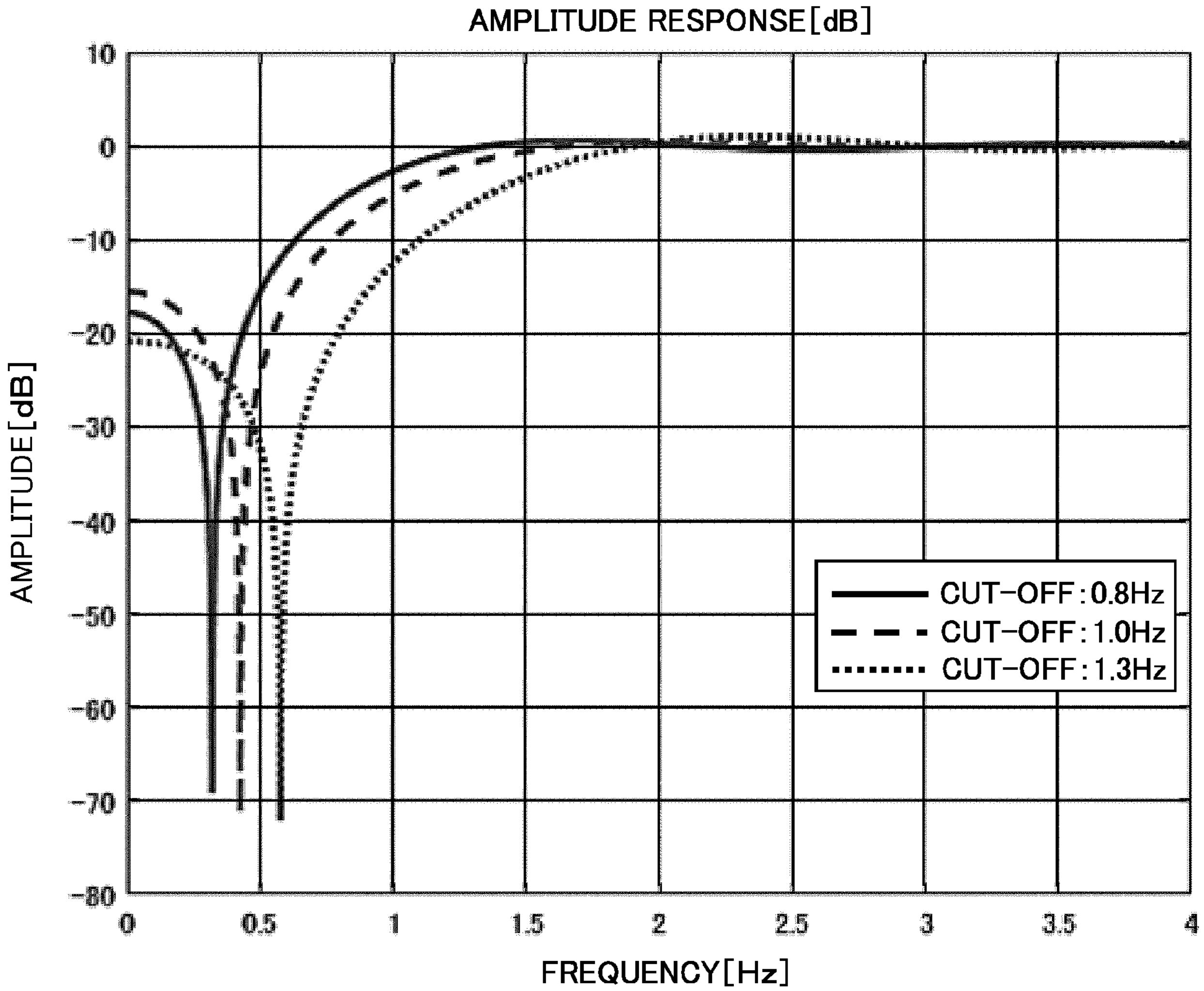
AMPLITUDE RESPONSE[dB]
10
0
-10
-20
-30
-40
-50
-60
-70
-80
AMPLITUDE[dB]
0
0.5
1
1.5
2
2.5
3
3.5
4
FREQUENCY[Hz]
CUT-OFF:0.8Hz
CUT-OFF:1.0Hz
CUT-OFF:1.3Hz

FIG. 7

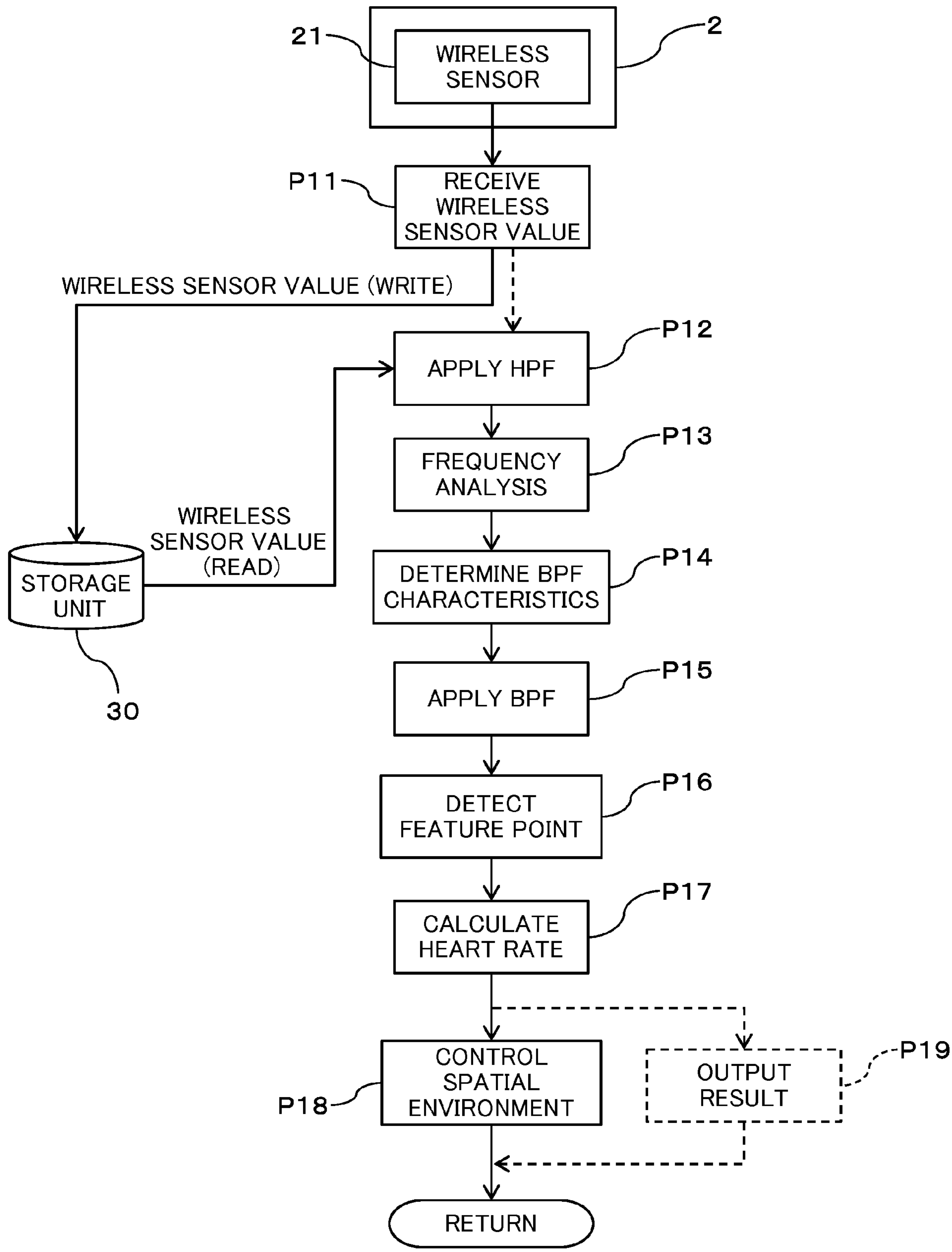
21
2
WIRELESS SENSOR
P11
RECEIVE WIRELESS SENSOR VALUE
WIRELESS SENSOR VALUE (WRITE)
P12
APPLY HPF
P13
FREQUENCY ANALYSIS
WIRELESS SENSOR VALUE (READ)
STORAGE UNIT
30
P14
DETERMINE BPF CHARACTERISTICS
P15
APPLY BPF
P16
DETECT FEATURE POINT
P17
CALCULATE HEART RATE
CONTROL SPATIAL ENVIRONMENT
P18
OUTPUT RESULT
P19
RETURN

FIG. 8

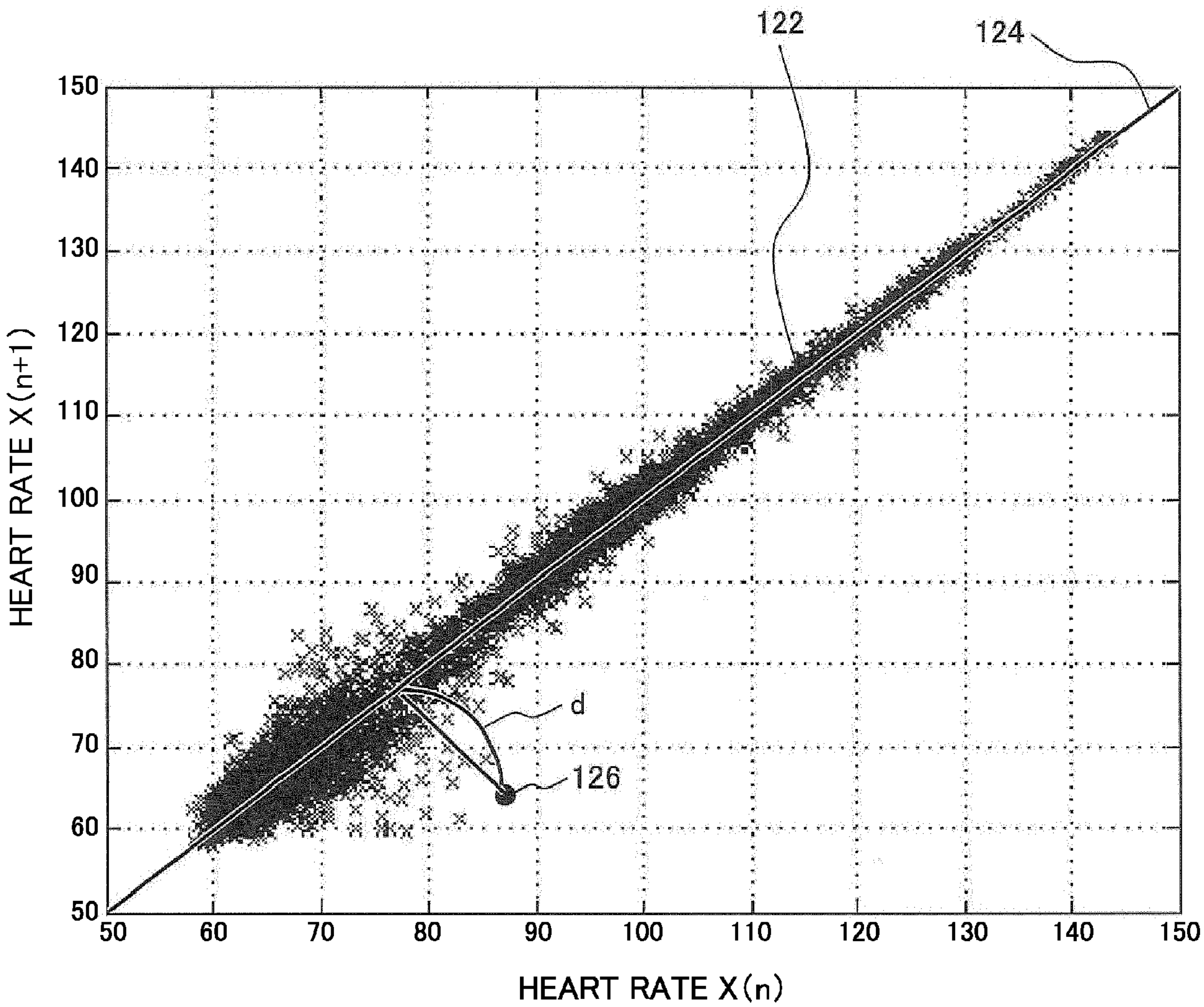
122
124
150
140
130
120
110
100
90
80
70
60
50
d
126
50 60 70 80 90 100 110 120 130 140 150
HEART RATE X(n+1)
HEART RATE X(n)

FIG. 9

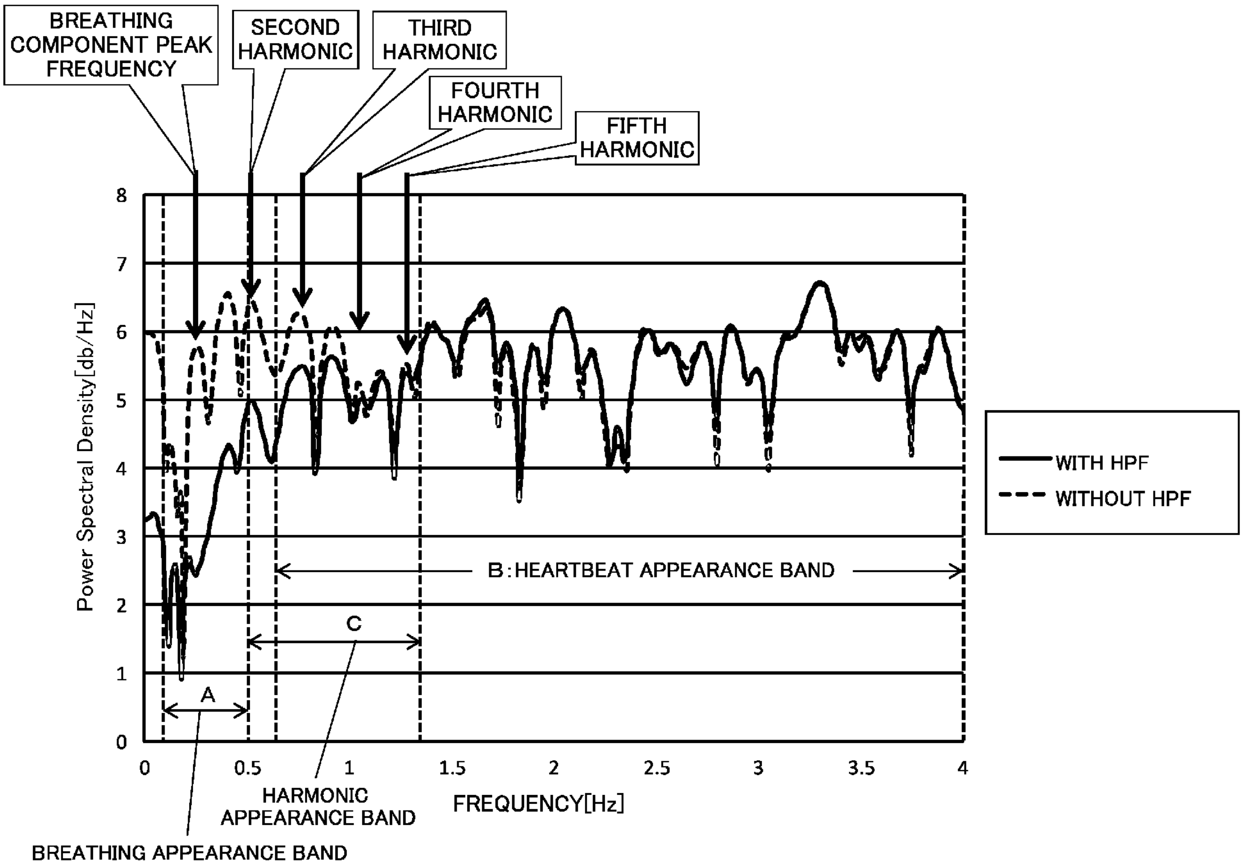
BREATHING COMPONENT PEAK FREQUENCY
SECOND HARMONIC
THIRD HARMONIC
FOURTH HARMONIC
FIFTH HARMONIC
8
7
6
5
4
3
2
1
0
Power Spectral Density[db/Hz]
WITH HPF
WITHOUT HPF
B: HEARTBEAT APPEARANCE BAND
C
A
0
0.5
1
1.5
2
2.5
3
3.5
4
HARMONIC APPEARANCE BAND
FREQUENCY[Hz]
BREATHING APPEARANCE BAND

FIG. 10

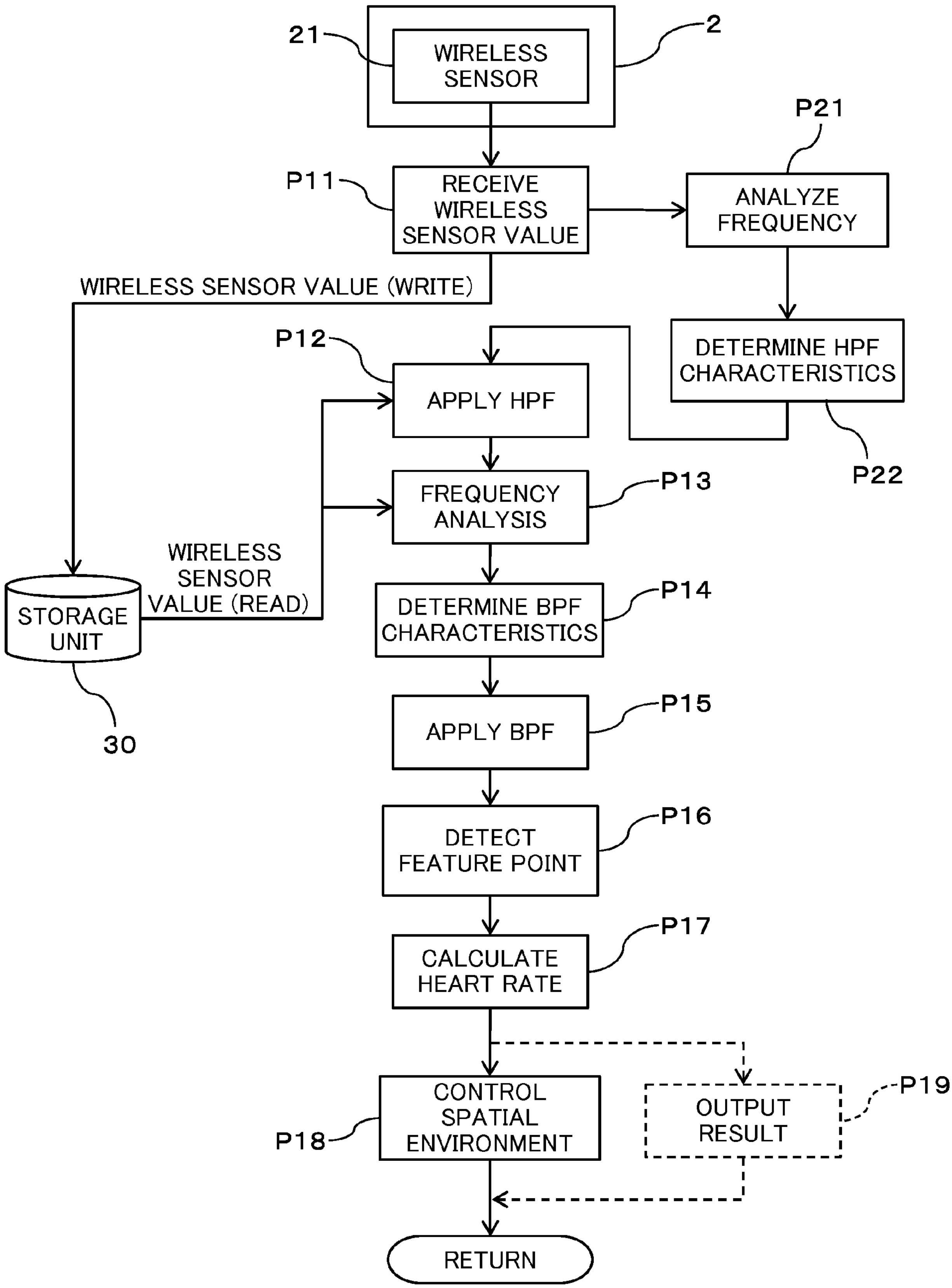

WIRELESS SENSOR
21
2
RECEIVE WIRELESS SENSOR VALUE
P11
ANALYZE FREQUENCY
P21
WIRELESS SENSOR VALUE (WRITE)
DETERMINE HPF CHARACTERISTICS
P22
APPLY HPF
P12
FREQUENCY ANALYSIS
P13
WIRELESS SENSOR VALUE (READ)
STORAGE UNIT
30
DETERMINE BPF CHARACTERISTICS
P14
APPLY BPF
P15
DETECT FEATURE POINT
P16
CALCULATE HEART RATE
P17
CONTROL SPATIAL ENVIRONMENT
P18
OUTPUT RESULT
P19
RETURN

FIG. 11

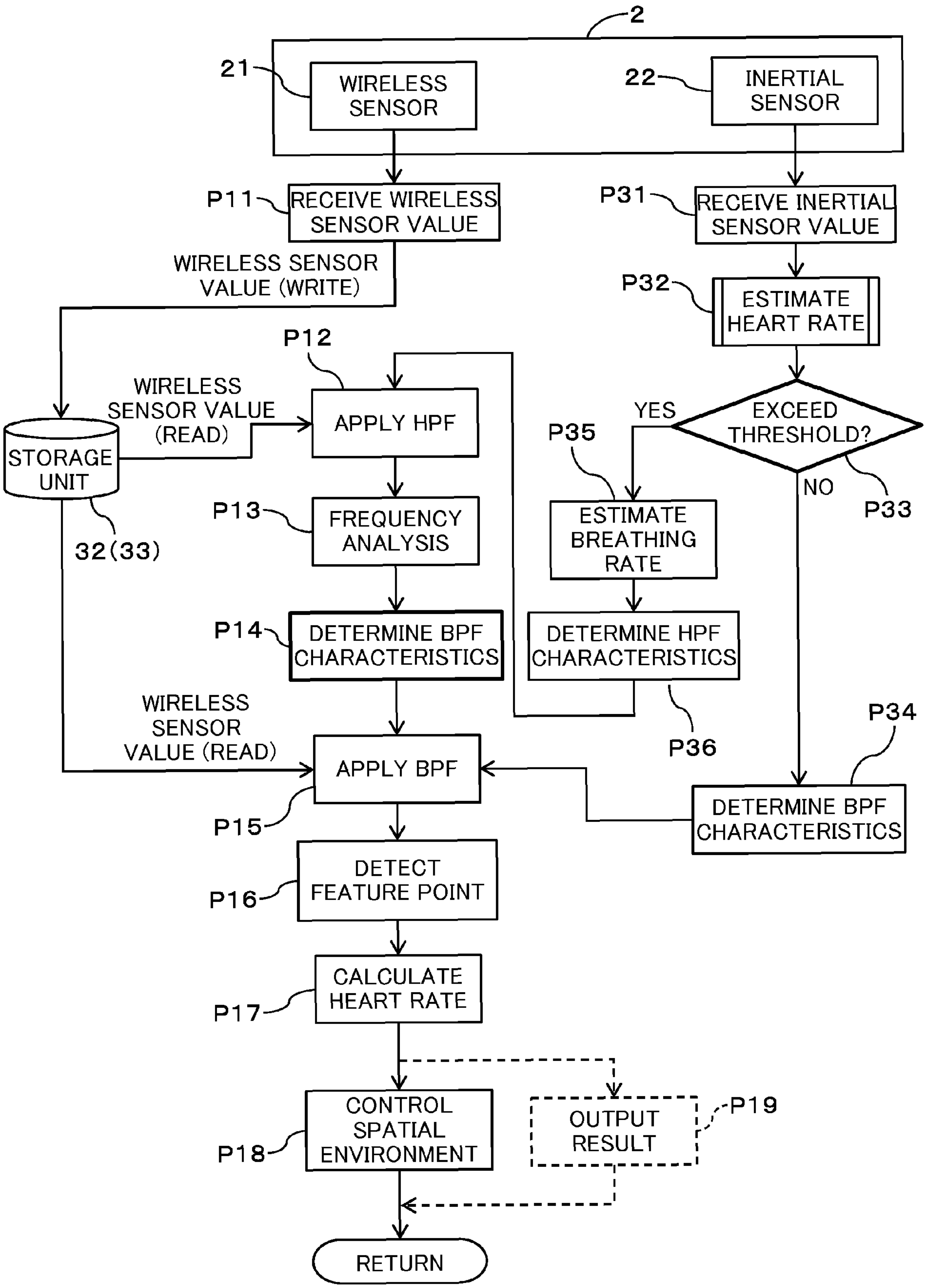
2
21
WIRELESS SENSOR
22
INERTIAL SENSOR
P11
RECEIVE WIRELESS SENSOR VALUE
P31
RECEIVE INERTIAL SENSOR VALUE
WIRELESS SENSOR VALUE (WRITE)
P32
ESTIMATE HEART RATE
P12
WIRELESS SENSOR VALUE (READ)
APPLY HPF
YES
EXCEED THRESHOLD?
P35
STORAGE UNIT
NO
P33
32(33)
P13
FREQUENCY ANALYSIS
ESTIMATE BREATHING RATE
P14
DETERMINE BPF CHARACTERISTICS
DETERMINE HPF CHARACTERISTICS
WIRELESS SENSOR VALUE (READ)
P34
P36
APPLY BPF
DETERMINE BPF CHARACTERISTICS
P15
DETECT FEATURE POINT
P16
CALCULATE HEART RATE
P17
CONTROL SPATIAL ENVIRONMENT
OUTPUT RESULT
P19
P18
RETURN

FIG. 12

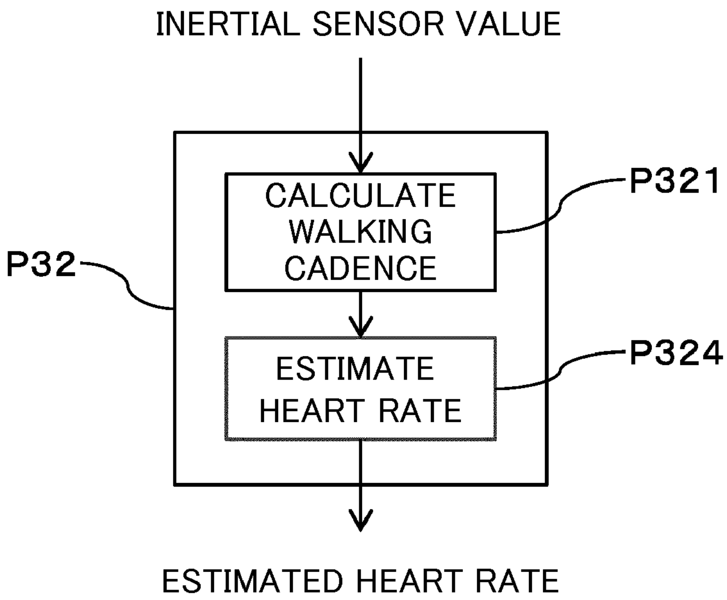
INERTIAL SENSOR VALUE
CALCULATE WALKING CADENCE
P321
P32
ESTIMATE HEART RATE
P324
ESTIMATED HEART RATE

FIG. 13

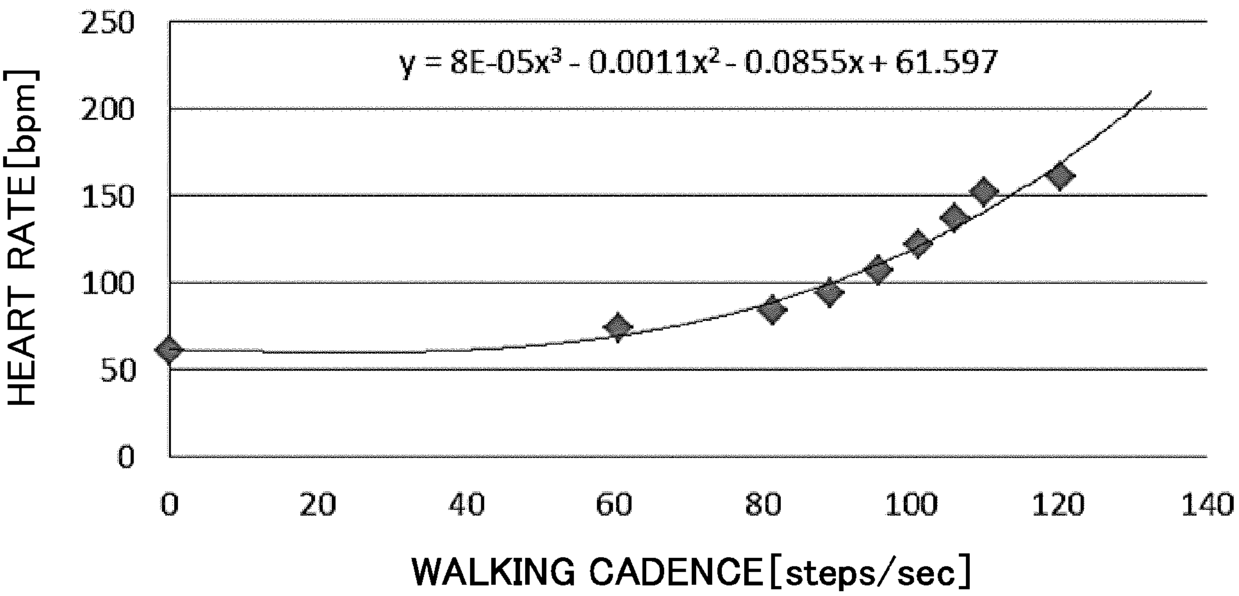
y = 8E-05x³ - 0.0011x² - 0.0855x + 61.597
HEART RATE [bpm]
250
200
150
100
50
0
0
20
40
60
80
100
120
140
WALKING CADENCE [steps/sec]

FIG. 14

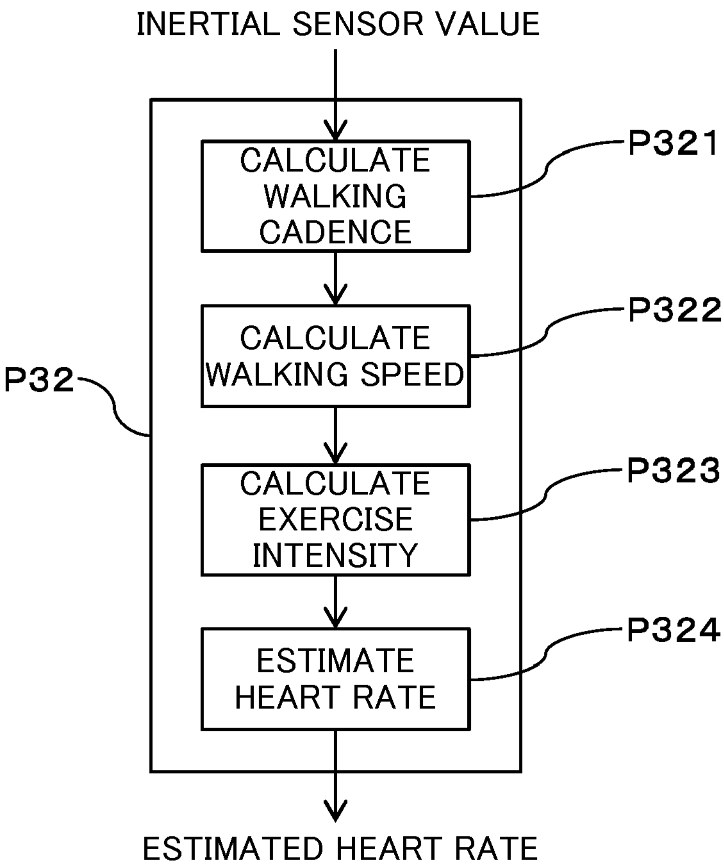
INERTIAL SENSOR VALUE
CALCULATE WALKING CADENCE
P321
CALCULATE WALKING SPEED
P322
P32
CALCULATE EXERCISE INTENSITY
P323
ESTIMATE HEART RATE
P324
ESTIMATED HEART RATE

FIG. 15

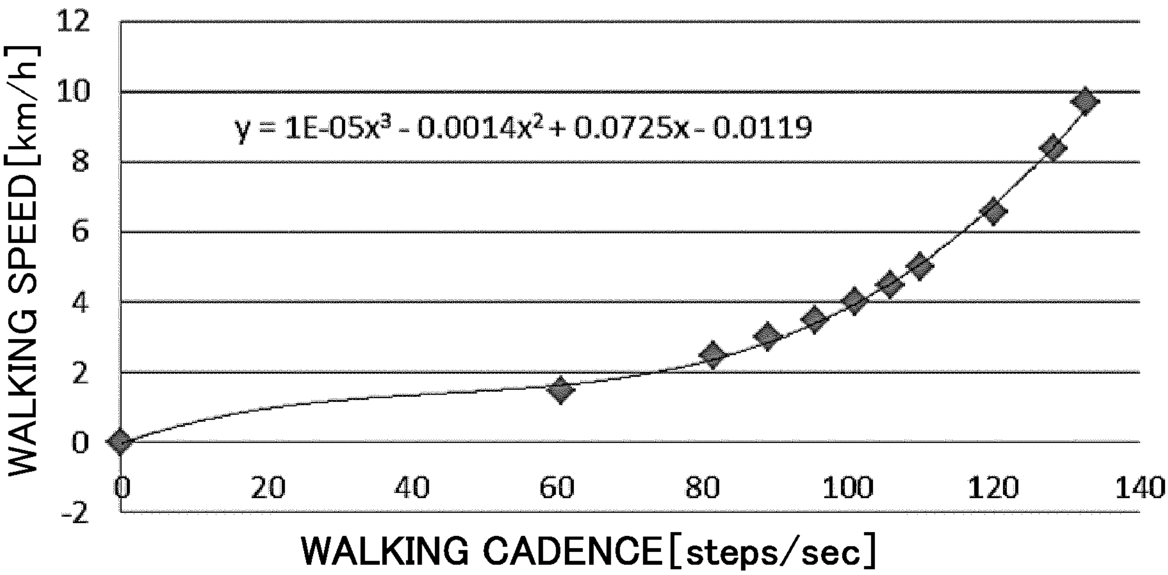
WALKING SPEED[km/h]
12
10
8
6
4
2
0
-2
y = 1E-05x³ - 0.0014x² + 0.0725x - 0.0119
0
20
40
60
80
100
120
140
WALKING CADENCE[steps/sec]

FIG. 16

| METs | km/h |
|---|---|
| 1.0 | 0.0 |
| 1.5 | 1.5 |
| 3.0 | 2.5 |
| 3.3 | 3.0 |
| 3.8 | 3.5 |
| 5.0 | 4.0 |
| 6.3 | 4.5 |
| 7.0 | 5.0 |
| 8.0 | 6.6 |
| 9.0 | 8.4 |
| 10.0 | 9.7 |
| 11.0 | 10.8 |
| 15.0 | 14.5 |

FIG. 17

| WALKING SPEED[Km/h] | BREATHING RATE[f/min] | HEART RATE[bpm] |
|---|---|---|
| 0 | 14.7 | 84 |
| 2 | 22.2 | 91 |
| 4 | 24 | 102 |
| 6 | 30.8 | 128 |
| 8 | 41 | 169 |
| 10 | 47.2 | 189 |

FIG. 18

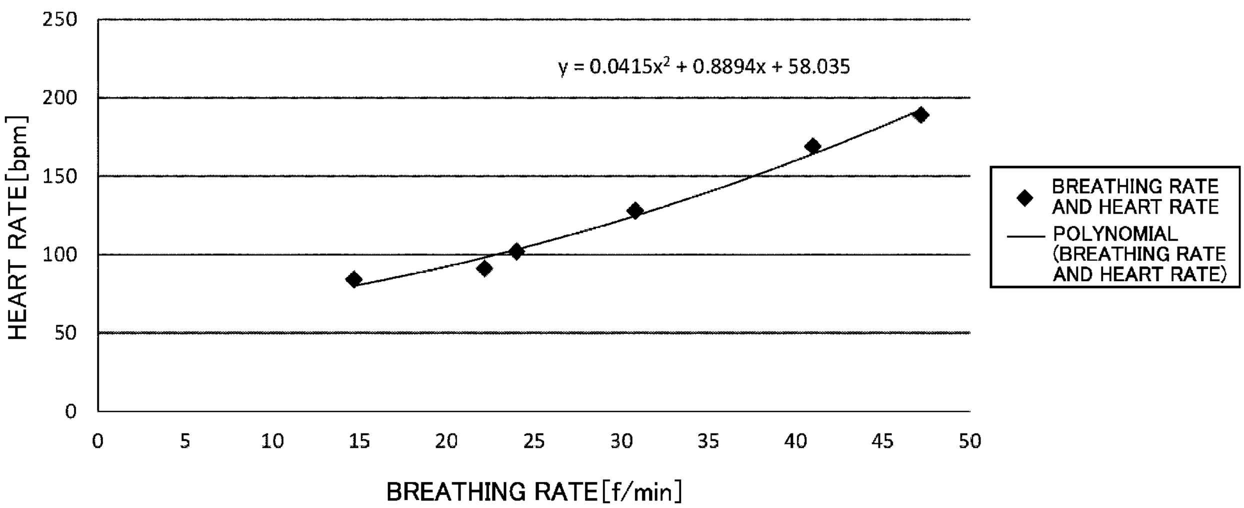
250
200
150
100
50
0
HEART RATE[bpm]
y = 0.0415x² + 0.8894x + 58.035
0
5
10
15
20
25
30
35
40
45
50
BREATHING RATE[f/min]
BREATHING RATE AND HEART RATE
POLYNOMIAL (BREATHING RATE AND HEART RATE)

FIG. 19

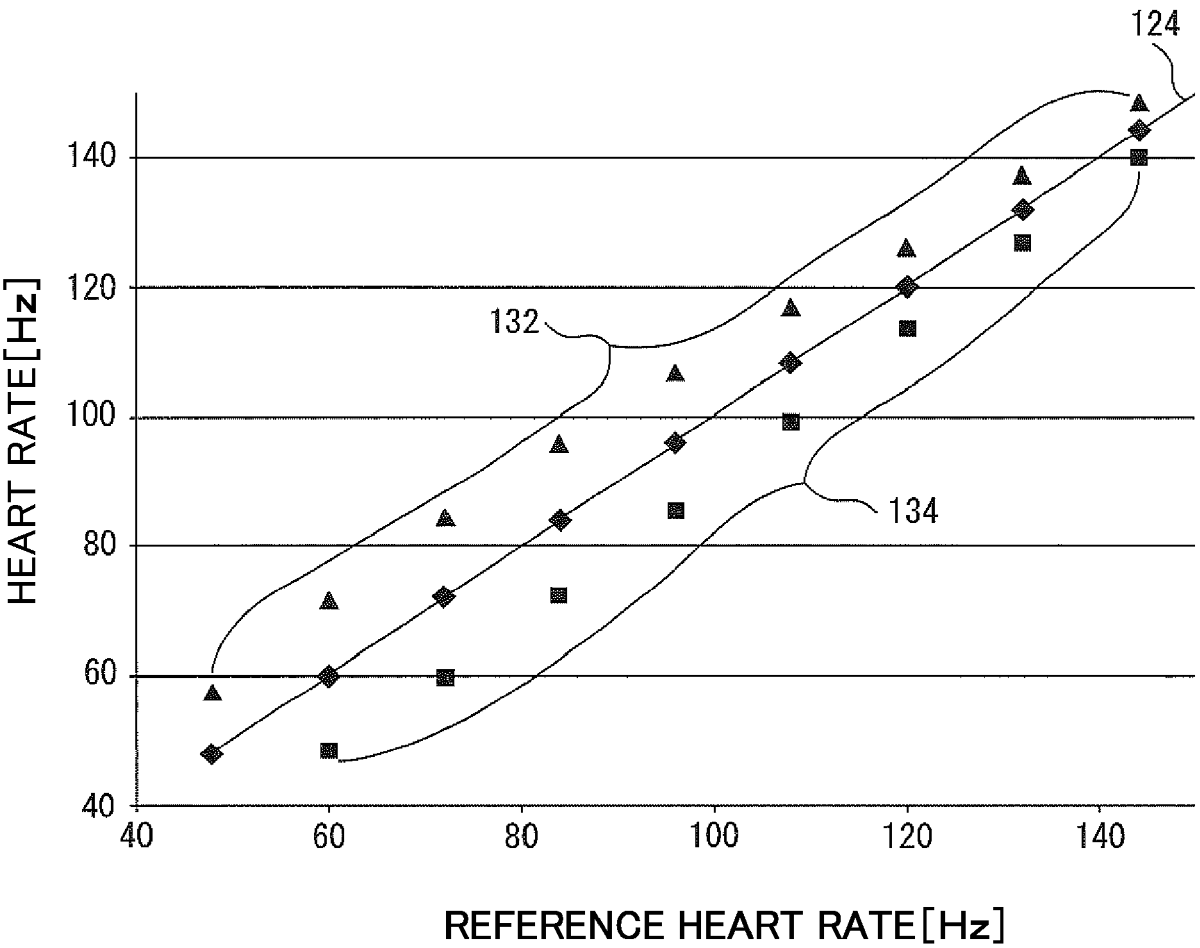
124
132
134
HEART RATE[Hz]
140
120
100
80
60
40
40
60
80
100
120
140
REFERENCE HEART RATE[Hz]

FIG. 20

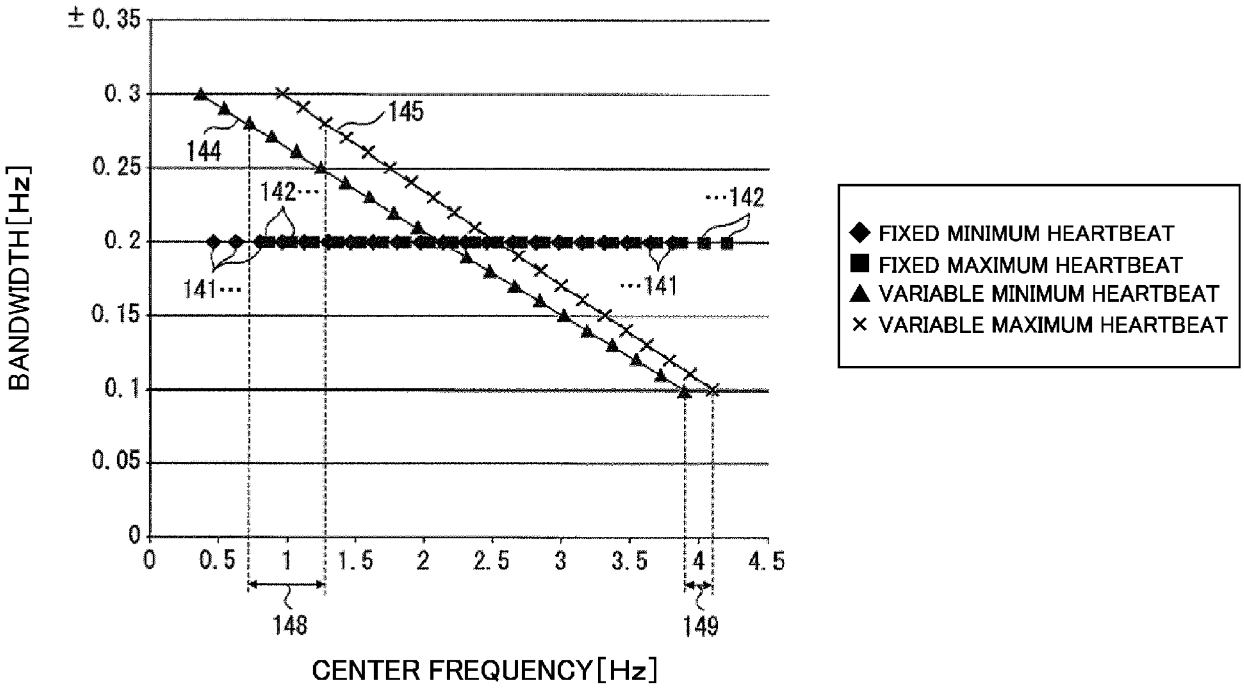
±0.35
0.3
0.25
0.2
0.15
0.1
0.05
0
BANDWIDTH[Hz]
0
0.5
1
1.5
2
2.5
3
3.5
4
4.5
CENTER FREQUENCY[Hz]
144
145
142…
…142
141…
…141
148
149
FIXED MINIMUM HEARTBEAT
FIXED MAXIMUM HEARTBEAT
VARIABLE MINIMUM HEARTBEAT
VARIABLE MAXIMUM HEARTBEAT

FIG. 21

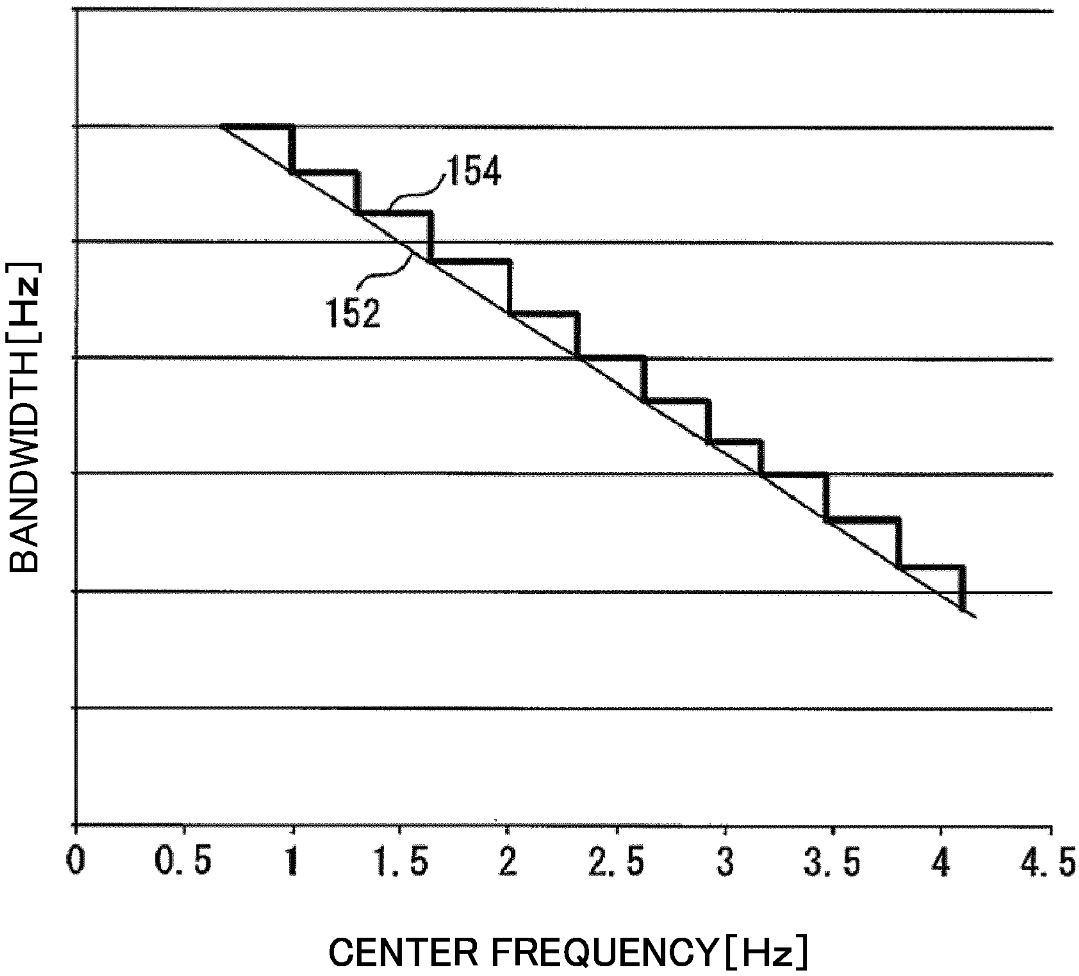

FIG. 22

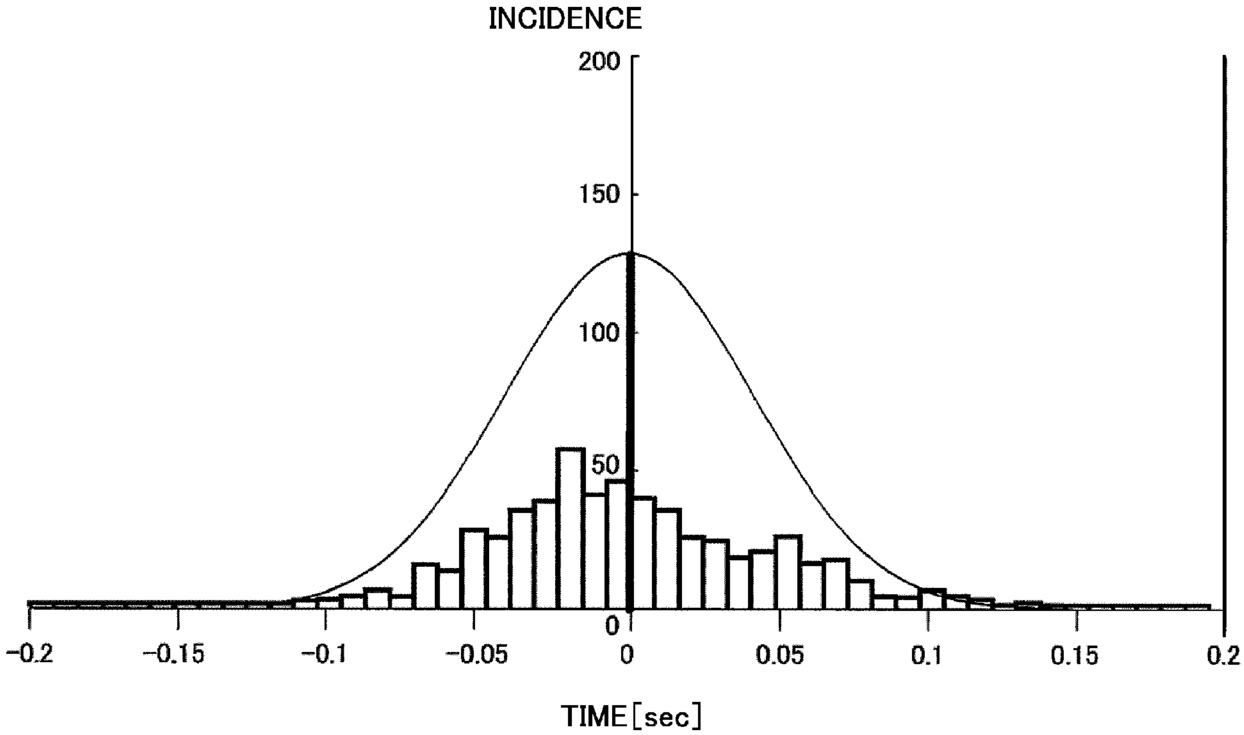

FIG. 23

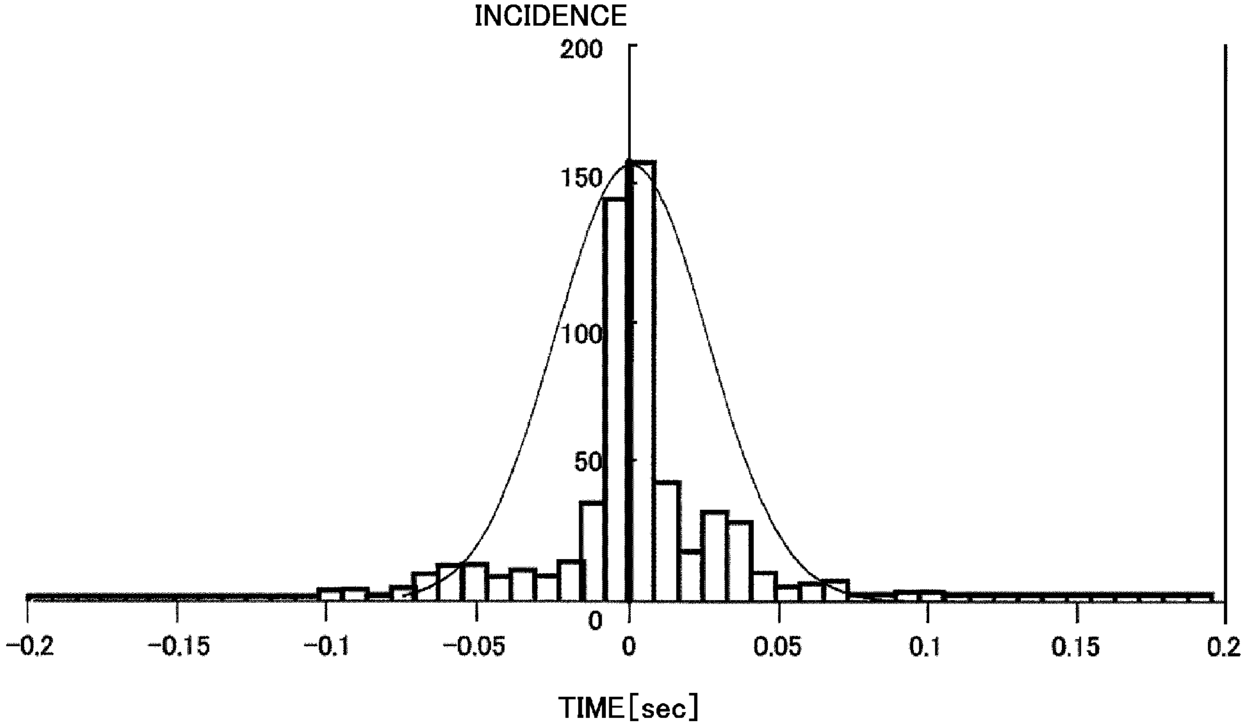

FIG. 24

| | HEART RATE NEAR 60 | HEART RATE NEAR 120 |
|---|---|---|
| THE NUMBER OF SAMPLES | 532 | 532 |
| AVERAGE VALUE Xbar | −0.000652964 | 0.000556391 |
| SAMPLE STANDARD DEVIATION $\sigma$ | 0.041438026 | 0.025446051 |
| MINIMUM VALUE Min | −0.10593357 | −0.103 |
| MAXIMUM VALUE Max | 0.134123404 | 0.096 |
| RANGE R | 0.240056974 | 0.199 |
| SKEW | 0.383 | −0.571 |
| KURTOSIS | −0.097 | 2.991 |
| BPF LOWER LIMIT | −0.165 | −0.165 |
| BPF UPPER LIMIT | 0.165 | 0.165 |
| CP | 1.33 | 2.16 |
| CPK | 1.32 | 2.15 |

FIG. 25

| | HEART RATE NEAR 60 | HEART RATE NEAR 120 |
|---|---|---|
| THE NUMBER OF SAMPLES | 532 | 532 |
| AVERAGE VALUE Xbar | −0.000652964 | 0.000556391 |
| SAMPLE STANDARD DEVIATION σ | 0.041438026 | 0.025446051 |
| MINIMUM VALUE Min | −0.10593357 | −0.103 |
| MAXIMUM VALUE Max | 0.134123404 | 0.096 |
| RANGE R | 0.240056974 | 0.199 |
| SKEW | 0.383 | −0.571 |
| KURTOSIS | −0.097 | 2.991 |
| BPF LOWER LIMIT | −0.1015 | −0.1015 |
| BPF UPPER LIMIT | 0.1015 | 0.1015 |
| CP | 0.82 | 1.33 |
| CPK | 0.81 | 1.32 |

FIG. 26

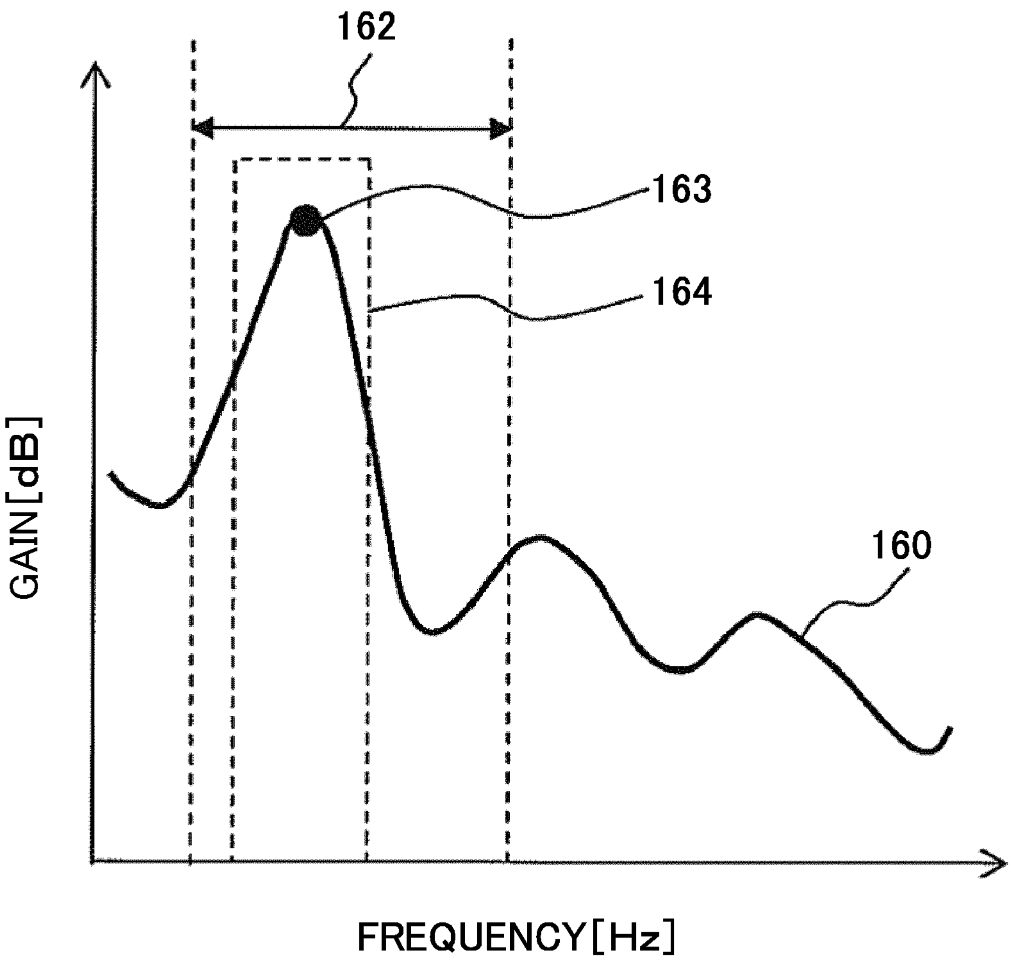

FIG. 27

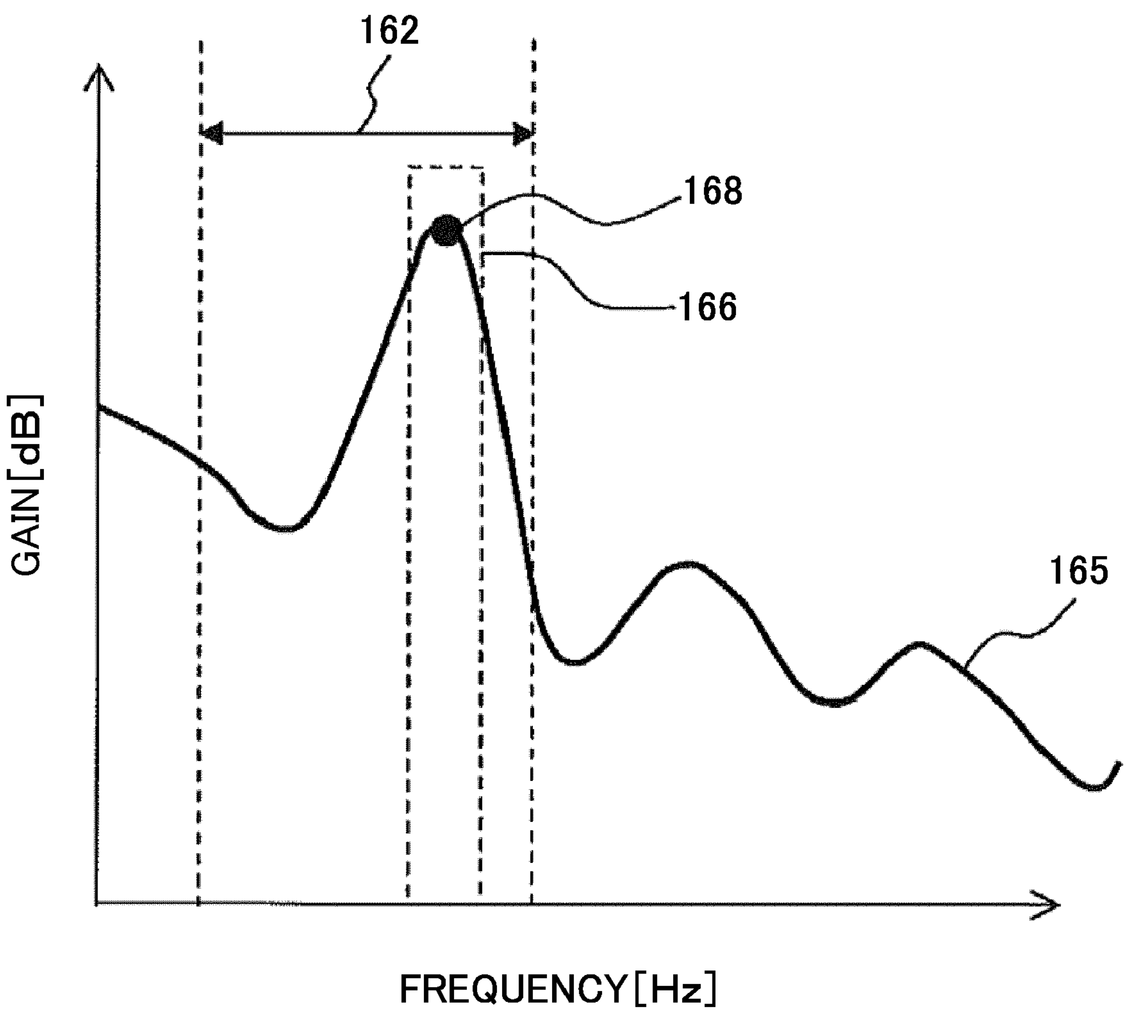

FIG. 28

BANDWIDTH INFORMATION170

171 172 173

| REFERENCE HEART RATE[bpm] | BPF WIDTH LOWER LIMIT[Hz] | BPF WIDTH UPPER LIMIT[Hz] |
|---|---|---|
| 40 | 0.366666667 | 0.966666667 |
| 50 | 0.543333333 | 1.123333333 |
| 60 | 0.72 | 1.28 |
| 70 | 0.896666667 | 1.436666667 |
| 80 | 1.073333333 | 1.593333333 |
| 90 | 1.25 | 1.75 |
| 100 | 1.426666667 | 1.906666667 |
| 110 | 1.603333333 | 2.063333333 |
| 120 | 1.78 | 2.22 |

Europäisches Patentamt
European Patent Office
Office européen des brevets

# EUROPEAN SEARCH REPORT

Application Number
EP 17 15 3710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/197377 A1 (KISHI TAKAHIKO [JP] ET AL) 1 August 2013 (2013-08-01)<br>* paragraph [0025] *<br>* paragraph [0040] - paragraph [0054] *<br>* figures 2A, 3 *<br>----- | 1-16 | INV.<br>A61B5/024<br>A61B5/08<br>A61B5/113<br>A61B5/00 |
| X | US 2007/118054 A1 (PINHAS ITZHAK [IL] ET AL) 24 May 2007 (2007-05-24)<br>* paragraph [0071] *<br>* paragraph [0105] *<br>* paragraph [0193] - paragraph [0198] *<br>* paragraph [0390] - paragraph [0391] *<br>----- | 1-16 | |
| X | LAZARO A ET AL: "Analysis of vital signs monitoring using an IR-UWB radar", PROGRESS IN ELECTROMAGNETICS RESEARCH. PIERS,,<br>vol. 100, 1 January 2010 (2010-01-01), pages 265-284, XP002593852,<br>* section 4 *<br>* figure 1 *<br>----- | 1,2,13, 15 | |

| TECHNICAL FIELDS SEARCHED (IPC) |
|---|
| A61B |

The present search report has been drawn up for all claims

1

| Place of search | Date of completion of the search | Examiner |
|---|---|---|
| The Hague | 13 July 2017 | Gooding Arango, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 17 15 3710

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2017

| Patent document cited in search report | Publication date | Patent family member(s) | Publication date |
|---|---|---|---|
| US 2013197377 A1 | 01-08-2013 | JP 5454593 B2 | 26-03-2014 |
| | | JP 2013153782 A | 15-08-2013 |
| | | US 2013197377 A1 | 01-08-2013 |
| US 2007118054 A1 | 24-05-2007 | CA 2668602 A1 | 10-05-2007 |
| | | EP 1955233 A2 | 13-08-2008 |
| | | JP 5281406 B2 | 04-09-2013 |
| | | JP 2009532072 A | 10-09-2009 |
| | | JP 2013154190 A | 15-08-2013 |
| | | US 2007118054 A1 | 24-05-2007 |
| | | US 2013245502 A1 | 19-09-2013 |
| | | WO 2007052108 A2 | 10-05-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014039666 A
- JP 1115344 A
- JP 2011115459 A
- JP 2008125595 A